# EUROPEAN PATENT APPLICATION

(11) **EP 3 757 197 A1**
(43) Date of publication of application: **30.12.2020**
(21) Application number: 19181997.8
(22) Date of filing: 24.06.2019
(51) Int. Cl.: C11D 3/20, C11D 3/50, A61K 8/33, A61Q 5/00, A61Q 19/00, C07C 43/03

(54) **1-ALKOXYETHYL-4-ISOBUTYL-BENZENE AS AROMA INGREDIENTS**

(71) Applicant: BASF SE, 67056 Ludwigshafen am Rhein (DE)
(72) Inventor: Pelzer, Ralf, 68623 Lampertheim (DE); Siegel, Wolfgang, 67056 Ludwigshafen (DE); Bru Roig, Miriam, 68623 Lampertheim (DE); Danz, Manuel, 67056 Ludwigshafen (DE); Weyland, Silke, 68623 Lampertheim (DE); Garlichs, Florian, 68623 Lampertheim (DE); Hickmann, Volker, 67056 Ludwigshafen (DE); Weingarten, Melanie, 67056 Ludwigshafen (DE)
(74) Representative: BASF IP Association

(57) **Abstract**

The presently claimed invention is directed to the use of 1-alkoxyethyl-4-isobutyl-benzene of formula (I) to impart an aroma impression to a composition. The presently claimed invention also relates to a method of imparting such aroma impression.

The present invention is further directed to compositions comprising the compound of formula (I) and at least one aroma chemical as well as to compositions comprising the compound of formula (I) and at least one further component selected from the group consisting of aroma chemicals, surfactants, oil components, anti-oxidants, deodorant-active agents and solvents.

The present invention also relates to a compound of formula (I').

## Description

### Field of the Invention

The presently claimed invention is directed to the use of 1-alkoxyethyl-4-isobutyl-benzene of formula (I) to impart an aroma impression to a composition. The presently claimed invention also relates to a method of imparting such aroma impression. The present invention is further directed to compositions comprising the compound of formula (I) and at least one aroma chemical as well as to compositions comprising the compound of formula (I) and at least one further component selected from the group consisting of aroma chemicals, surfactants, oil components, anti-oxidants, deodorant-active agents and solvents. The present invention also relates to a compound of formula (I').

### Background of the invention

Aroma chemicals, especially fragrances, are of great interest in the field of cosmetics and cleaning and laundry compositions. Fragrances of natural origin are mostly expensive, often limited in theiravailablity and, on account of fluctuations in environmental conditions, are also subject to variations in their content, purity etc. To circumvent these undesirable factors, it is therefore of great interest to create synthetic substances which have sensory properties that resemble more expensive natural fragrances, or which have novel and interesting sensory profiles.

Despite a large number of already existing aroma chemicals, there is a constant need for new components in order to be able to satisfy the multitude of properties desired for extremely diverse areas of application. These include, firstly, the sensory properties, i.e. the compounds should have advantageous odiferous (olfactory) or gustatory properties. Furthermore, aroma chemicals should also have additional positive secondary properties, such as e.g. an efficient preparation method, the possibility of providing better sensory profiles as a result of synergistic effects with other aroma chemicals, a higher stability in a wide range of compositions as well as under certain application conditions, a higher extendibility and/or a better staying power.

There is an increased need for aroma chemicals which can impart an aroma impression, especially a flowery and/or a marine and/or a green odiferous impression to a composition. Such properties are of special interest for compositions such as for example body care compositions, hygiene articles, cleaning compositions, textile detergent compositions and compositions for scent dispensers. Of special interest are aroma chemicals, which can impart one or more distinct aroma impressions to a composition, thereby contributing to a rich and interesting sensory profile, especially an olfactory profile of the composition. In addition, especially regarding aroma chemicals which can impart an aroma impression, the substantivity as well as the tenacity are of special interest in order to obtain a long-lasting odiferous impression in the composition as well as to the surface with which the composition is treated.

It is an object of the present invention to provide an aroma chemical, which can impart an aroma impression, a flowery and/or a marine and/or a green and/or a sweet note and/or a rubbery note and/or a nutty note and/or a woody note and/or a dusty note and/or a rooty note and/or a lemon note odiferous impression, to a composition. It is further an object of the present invention to provide an aroma chemical, which can impart a long-lasting aroma impression, especially a flowery and/or a marine and/or a green and/or a sweet note and/or a rubbery note and/or a nutty note and/or a woody note and/or a dusty note and/or a rooty note and/or a lemon note odiferous impression, to a composition as well as to surfaces treated with such compositions, such as for example skin and or textiles and is stable in a wide variety of compositions.

### Summary of the Invention

It was surprisingly found that a compound of formula (I) imparts an aroma impression, especially a flowery and/or a marine and/or a green and/or a sweet note and/or a rubbery note and/or a nutty note and/or a woody note and/or a dusty note and/or a rooty note and/or a lemon note odiferous impression, to a composition. Such an aroma impression is stable in the wide range of compositions and, thus, contributes to a rich and interesting aroma profile of the composition.

Thus, in one aspect the present invention is directed to the use of a compound of formula (I), 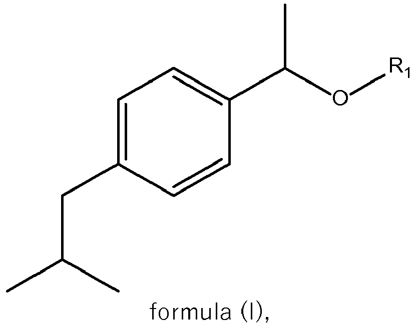 wherein
R₁ is selected from the group consisting of linear or branched, unsubstituted -C₁-C₅ alkyl and unsubstituted -C₃-C₅ cycloalkyl;
or its salt or stereoisomer thereof,
to impart an aroma impression to a composition.

In another aspect, the present invention provides a method of imparting an aroma impression to a composition comprising at least the step of adding a compound of formula (I), 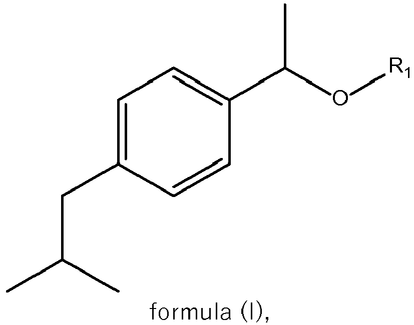 wherein
R₁ is selected from the group consisting of linear or branched, unsubstituted -C₁-C₅ alkyl and unsubstituted -C₃-C₅ cycloalkyl;
or its salt or stereoisomer thereof,
to a composition.

In yet another aspect, the present invention provides a composition comprising a at least one compound of the formula (I), 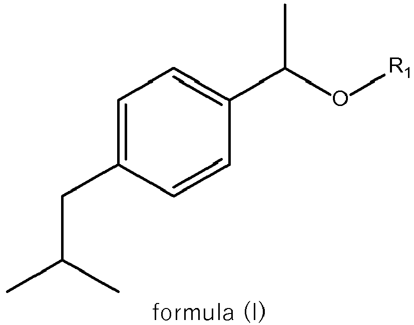 wherein R₁ is selected from the group consisting of linear or branched, unsubstituted -C₁-C₅ alkyl and unsubstituted -C₃-C₅ cycloalkyl;
or its salt or stereoisomer thereof and
(i) at least one aroma chemical, or
(ii) at least one non-aroma chemical carrier, or
(iii) both of (i) and (ii).

In yet another aspect, the present invention provides a compound of formula (I'), 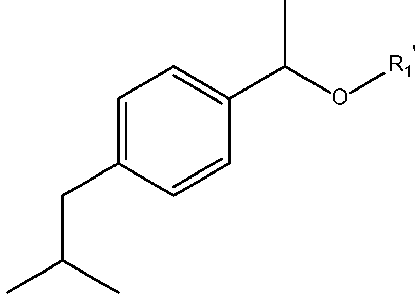 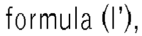 wherein
R₁' is selected from the group consisting of linear or branched -CH₂CH₂CH₃, -CH(CH₃)(C₂H₅), -CH₂CH(CH₃)₂ and -CH₂CH₂CH₂CH₃;
or its salt or stereoisomer thereof.

### Detailed Description of the Invention

The following detailed description is merely exemplary in nature and is not intended to limit the presently claimed invention or the application and uses of the presently claimed invention. Furthermore, there is no intention to be bound by any theory presented in the preceding technical field, background, summary or the following detailed description.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or AA), BB) and CC) or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" or "preferred embodiment" means that a particular feature, structure or characteristic described in connection with the embodiment is included in at least one embodiment of the presently claimed invention. Thus, appearances of the phrases "in one embodiment" or "In a preferred embodiment" or "in a preferred embodiment" in various places throughout this specification are not necessarily all referring to the same embodiment but may refer. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments are used in any combination.

Furthermore, the ranges defined throughout the specification include the end values as well, i.e. a range of 1 to 10 implies that both 1 and 10 are included in the range. For the avoidance of doubt, the applicant shall be entitled to any equivalents according to applicable law.

### Definitions

In the context of the present invention, the term "aroma" refers to a sensory property and comprises an odor and/or a flavor.

The term "aroma chemical" denotes a substance which is used to obtain a sensory or organoleptic (used interchangeably herein) impression and comprises its use to obtain an olfactory and/or a flavor impression. The term "olfactory impression" denotes an odor impression without any positive or negative judgement, while the term "scent impression" or "fragrance impression" or "aroma impression" (used interchangeably herein) as used herein is connected to an odor impression which is generally felt as pleasant. Thus a "fragrance" or "scent" denotes an aroma chemical, which predominately induces a pleasant odor impression. A flavor induces a taste impression.

The term "aroma composition", as used herein, refers to a composition which induces an aroma. The term aroma composition comprises "odor composition" and/or "flavor composition". An odor composition being a composition, which predominately induces an odor impression, a flavor composition being a composition, which predominantly induces a taste impression.

The term odor composition comprises "fragrance composition" or "scent composition" (used interchangeably herein), which predominately induce an odor impression which is generally felt as pleasant.

The general hedonistic expressions "advantageous sensory properties" or "advantageous organoleptic properties" describe the niceness and conciseness of an organoleptic impression conveyed by an aroma chemical. "Niceness" and "conciseness" are terms which are familiar to the person skilled in the art, such as a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

The expressions "combination of", "in combination with" or "combined with" when used herein referring to the compositions, methods or the use of two compounds, take account of the fact that the two compounds do not need to be used in the form of a physical mixture of said compounds but can be used (e.g., added) separately. Where the compounds are used separately, they can be used (e.g. added) sequentially (i.e. one after the other) in any order, or concurrently (i.e. basically at the same time).

The term "boosting" or "boost" is used herein to describe the effect of enhancing and/or modifying the aroma of an aroma chemical or of a composition. The term "enhancing" comprises an improvement of the niceness and/or conciseness of an aroma and/or an improvement of the intensity. The term "modifying" comprises the change of an aroma profile.

The intensity can be determined via a threshold value determination. A threshold value of an odor is the concentration of a substance in the relevant gas space at which an odor impression can just still be perceived by a representative test panel, although it no longer has to be defined.

Booster effects are particularly desired in fragrance composition when top-note-characterized applications are required, in which the odor is to be conveyed particularly quickly and intensively, for example in deodorants, air fresheners or in the taste sector in chewing gums.

The terms "the invention relates to" and "the invention is directed to" are used synonymously throughout the invention.

The terms "compound" and "substance" are used synonymously throughout the invention.

The term "tenacity" describes the evaporation behavior over time of an aroma chemical. The tenacity can for example be determined by applying the aroma chemical to a test strip, and by subsequent olfactory evaluation of the odor impression of the test strip. For aroma chemicals with high tenacity the time span after which the panel can still identify an aroma impression is long.

The term "substantivity" describes the interaction of an aroma chemical with a surface, such as for example the skin or a textile, especially after subsequent treatment of the surface, such as for example washing. The substantivity can for example be determined by washing a textile with a textile detergent composition comprising the aroma chemical and subsequent olfactory evaluation of the textile directly after washing (wet textile) as well as evaluation of the dry textile after prolonged storage.

The term "stability" describes the behavior of an aroma chemical upon contact with oxygen, light and/or other substances. An aroma chemical with high stability maintains its aroma profile over a long period in time, preferably in a large variety of compositions and under various storage conditions.

In order to impart a long-lasting aroma impression to a composition or to a surface treated with a composition, the tenacity, the substantivity as well as the stability of the aroma chemical in the compositions should preferably be high.

### Use and method

One embodiment of the invention is directed to a use of a compound of formula (I), 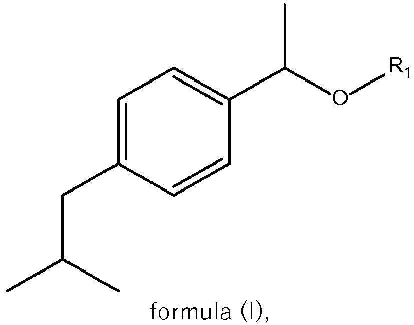 wherein
R₁ is selected from the group consisting of linear or branched, unsubstituted -C₁-C₅ alkyl and unsubstituted -C₃-C₅ cycloalkyl;
or its salt or stereoisomer thereof,
to impart an aroma impression to a composition.

One embodiment of the invention is directed to a method of imparting an aroma impression to a composition comprising at least the step of adding a compound of formula (I), 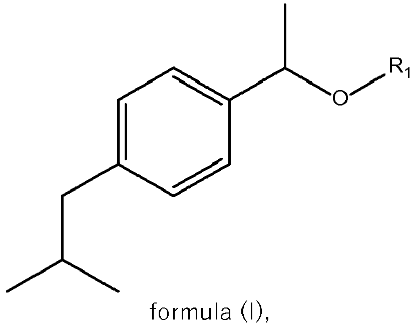 wherein
R₁ is selected from the group consisting of linear or branched, unsubstituted -C₁-C₅ alkyl and unsubstituted -C₃-C₅ cycloalkyl;
or its salt or stereoisomer thereof,
to a composition.

In a preferred embodiment, R₁ is selected from the group consisting of -CH₃, -CH₂CH₃,-CH₂CH₂CH₃, -CH(CH₃)₂, -CH(CH₃)(C₂H₅), -CH₂CH(CH₃)₂, -CH₂CH₂CH₂CH₃ and cyclopropyl, more preferably R₁ is selected from the group consisting of -CH₃, -CH₂CH₃, -CH(CH₃)₂,-CH(CH₃)(C₂H₅) and -CH₂CH(CH₃)₂.

In yet another preferred embodiment, the composition is selected from perfume compositions, body care compositions, hygiene articles, cleaning compositions, textile detergent compositions, compositions for scent dispensers, foods, food supplements, pharmaceutical compositions and crop protection compositions.

In one embodiment, the impression is a flowery note. In one embodiment, the impression is a marine note. In one embodiment, the impression is a green note. In one embodiment, the impression is a sweet note. In one embodiment, the impression is a rubbery note. In one embodiment, the impression is a nutty note. In one embodiment, the impression is a woody note. In one embodiment, the impression is a dusty note. In one embodiment, the impression is a rooty note. In one embodiment, the impression is a lemon note.

In a preferred embodiment, the compound of formula (I) or its salts or stereoisomer is present in an amount in the range of ≥ 0.01 wt.% to ≤ 70.0 wt.%, more preferably in the range of ≥ 0.05 wt.% to ≤ 60.0 wt.%, particularly in the range of ≥ 0.1 wt.% to ≤ 50.0 wt.%, based on the total weight of the composition. In yet another preferred embodiment, the compound of formula (I) or its salts or stereoisomer is present in an amount in the range of 0.05 wt.% to 10 wt.%, more preferably 0.1 wt.% to 5 wt.%, yet more preferably 0.2 wt.% to 3 wt.%, most preferably 20 wt.% to 70 wt.%, particularly 25 wt.% to 50 wt.%, based on the total weight of the composition.

### Composition

One embodiment of the presently claimed invention is directed to a composition comprising a at least one compound of the formula (I), 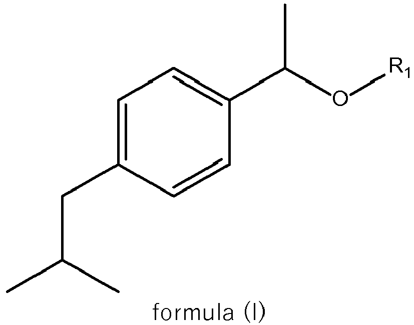 wherein
R₁ is selected from the group consisting of linear or branched, unsubstituted -C₁-C₅ alkyl and unsubstituted -C₃-C₅ cycloalkyl;
or its salt or stereoisomer thereof and
(i) at least one aroma chemical, or
(ii) at least one non-aroma chemical carrier, or
(iii) both of (i) and (ii).

In a preferred embodiment, R₁ is selected from the group consisting of -CH₃, -CH₂CH₃,-CH₂CH₂CH₃, -CH(CH₃)₂, -CH(CH₃)(C₂H₅), -CH₂CH(CH₃)₂, -CH₂CH₂CH₂CH₃, cyclopropyl and cyclobutyl. In yet another preferred embodiment, R₁ is selected from the group consisting of -CH₃, -CH₂CH₃, -CH₂CH₂CH₃, -CH(CH₃)₂, -CH(CH₃)(C₂H₅), -CH₂CH(CH₃)₂ and-CH₂CH₂CH₂CH₃. In yet another preferred embodiment, R₁ is selected from the group consisting of cyclopropyl and cyclobutyl.

In yet another preferred embodiment, the at least one aroma chemical (i) is selected from the group consisting of geranyl acetate, alpha-hexylcinnamaldehyde, 2-phenoxyethyl isobutyrate, dihydromyrcenol, methyl dihydrojasmonate , 4,6,6,7,8,8-hexamethyl-1,3,4,6,7,8-hexahydrocyclopenta[g]benzopyran, tetrahydrolinalool, ethyllinalool, benzyl salicylate, 2-methyl-3-(4-tert-butylphenyl)propanal, cinnamyl alcohol, 4,7-methano-3a,4,5,6,7,7a-hexahydro-5-indenyl acetate and/or 4,7-methano-3a,4,5,6,7,7a-hexahydro-6-indenyl acetate, citronellol, citronellyl acetate, tetrahydrogeraniol, vanillin, linalyl acetate, styrolyl acetate, octahydro-2,3,8,8-tetramethyl-2-acetonaphthone and/or 2-acetyl-1,2,3,4,6,7,8-octahydro-2,3,8,8-tetramethylnaphthalene, hexyl salicylate, 4-tert-butylcyclohexyl acetate, 2-tert-butylcyclohexyl acetate, alpha-ionone, n-alpha-methylionone, alpha-isomethylionone, coumarin, terpinyl acetate, 2-phenylethyl alcohol, 4-(4-hydroxy-4-methylpentyl)-3-cyclohexenecarboxaldehyde, alpha-amylcinnamaldehyde, ethylene brassylate, (E)- and/or (Z)-3-methylcyclopentadec-5-enone, 15-pentadec-11-enolide and/or 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3-(3-isopropylphenyl)butanal, 2-methyl-3-(3,4-methylenedioxyphenyl)propanal, 3-(4-ethylphenyl)-2,2-dimethylpropanal, 7-methyl-2H-1,5-benzodioxepin-3(4H)-one, 3,3,5-trimethylcyclohexyl acetate, 2,5,5-trimethyl-1,2,3,4,4a,5,6,7-octahydronaphthalen-2-ol, 3-(4-*tert*-butylphenyl)-propanal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate and 3-[5,5,6-trimethylbicyclo[2.2.1]hept-2-yl]cyclohexan-1-ol.

In yet another preferred embodiment, the at least one aroma chemical (i) is selected from the group consisting of methyl benzoate, benzyl acetate, geranyl acetate, 2-isobutyl-4-methyltetrahydro-2H-pyran-4-ol, linalool, 2-isobutyl-4-methyltetrahydro-2H-pyran-4-ol and methyl benzoate.

In yet another preferred embodiment, the at least one aroma chemical (i) is selected from the group consisting of ethylvanillin, vanillin, 2,5-dimethyl-4-hydroxy-2H-furan-3-one (furaneol) or 3-hydroxy-2-methyl-4H-pyran-4-one (maltol).

Further aroma chemicals with which the compound of formula (I) can be combined to give a composition according to the presently claimed invention can be found, e.g., in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3 carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1 octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10 trimethyl-5,9-undecadienal; heptanal diethylacetal; 1,1-dimethoxy-2,2,5 trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3 heptanone oxime; 2,4,4,7-tetramethyl-6-octen-3-one; 6-methyl-5-hepten-2-one;
the aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e.g. 2-nonenenitrile; 2-undecenenitrile; 2 tridecenenitrile; 3,12-tridecadienenitrile; 3,7-dimethyl-2,6-octadienenitrile; 3,7-dimethyl-6 octenenitrile;
the esters of aliphatic carboxylic acids such as e.g. (E) and (Z)-3-hexenyl formate; ethyl acetoacetate; isoamyl acetate; hexyl acetate; 3,5,5-trimethylhexyl acetate; 3 methyl-2-butenyl acetate; (E)-2-hexenyl acetate; (E) and (Z)-3-hexenyl acetate; octyl acetate; 3-octyl acetate; 1-octen-3-yl acetate; ethyl butyrate; butyl butyrate; isoamyl butyrate; hexyl butyrate; (E) and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2 ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2 butenoates thereof;
the acyclic terpene aldehydes and ketones such as e.g. geranial; neral; citronellal; 7 hydroxy-3,7-dimethyloctanal; 7 methoxy-3,7-dimethyloctanal; 2,6,10-trimethyl-9 undecenal; geranyl acetone; as well as the dimethyl and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8 mercap-tomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano¬naphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1 (4-isopropylcyclohexyl)ethanol; 2-methyl-4-(2,2,3-trimethyl-3-cyclopent-1-yl)butanol; 2-methyl-4-(2,2,3 trimethyl-3-cyclopent-1-yl)-2-buten-1-yl; 2-ethyl-4-(2,2,3-trimethyl-3 cyclopent-1-yl)-2-buten-1-ol; 3-methyl-5-(2,2,3 trimethyl-3-cyclopent-1-yl)pentan-2 ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclo-dodecane; alpha-cedrene epoxide; 3a,6,6,9a-tetramethyldodecahydronaphtho[2,1-b]furan; 3a-ethyl-6,6,9a-trimethyldodecahydro-naphtho[2,1-b]furan; 1,5,9-trimethyl-13-oxabicyclo- [10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5 trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2 cyclopenten-1-one; 3-methyl-2-pentyl-2-cyclopenten-1-one; 3-methyl-4-cyclopenta-decenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclo-hexadecen-1-one; 7-cyclohexadecen-1-one; (7/8)-cyclohexadecen-1-one; 9 cyclo-heptadecen-1-one; cyclopentadecanone; cyclohexadecanone;
the cycloaliphatic aldehydes such as e.g. 2,4-dimethyl-3-cyclohexenecarbaldehyde; 2 methyl-4-(2,2,6-trimethylcyclohexen-1-yl)-2-butenal; 4-(4-hydroxy-4-methylpentyl)-3 cyclohexene carbaldehyde; 4-(4-methyl-3-penten-1-yl)-3-cyclohexenecarbaldehyde;
the cycloaliphatic ketones such as e.g. 1-(3,3-dimethylcyclohexyl)-4-penten-1-one; 2,2 dimethyl-1-(2,4-dimethyl-3-cyclohexen-1-yl)-1-propanone; 1-(5,5-dimethyl-1 cyclo-hexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6 indenyl propionate; 4,7-methano-3a,4,5,6,7,7a-hexahydro-5 or 6-indenyl isobutyrate; 4,7 methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis and trans-methyl dihydrojasmonate; cis and trans-methyl jasmonate; methyl 2-hexyl-3-oxocyclopentanecarboxylate; ethyl 2-ethyl-6,6 dimethyl-2-cyclohexenecarboxylate; ethyl 2,3,6,6-tetramethyl-2 cyclohexene-carboxylate; ethyl 2-methyl-1,3-dioxolane-2-acetate;
the araliphatic alcohols such as e.g. benzyl alcohol; 1-phenylethyl alcohol, 2 phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2 phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxy-,benzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2 phenylethyl isovalerate; 1 phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2 phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m dioxine;
the aromatic and araliphatic aldehydes such as e.g. benzaldehyde; phenylacetaldehyde; 3-phenylpropanal; hydratropaaldehyde; 4-methylbenzaldehyde; 4 methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3 methyl-5-phenylpentanal; 4-methoxybenzaldehyde; 4-hydroxy-3 methoxy-benzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzo¬phenone; 1,1,2,3,3,6-hexamethyl-5-indanyl methyl ketone; 6-tert-butyl-1,1 dimethyl-4 indanyl methyl ketone; 1-[2,3-dihydro-1,1,2,6-tetramethyl-3-(1-methylethyl)-1H-5 indenyl]ethanone; 5',6',7',8'-tetrahydro-3',5',5',6',8',8'-hexamethyl-2-acetonaphthone;
the aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
the nitrogen-containing aromatic compounds such as e.g. 2,4,6-trinitro-1,3-dimethyl-5 tert-butylbenzene; 3,5-dinitro-2,6-dimethyl-4-tert-butylacetophenone; cinnamonitrile; 3 methyl-5-phenyl-2-pentenonitrile; 3-methyl-5-phenylpentanonitrile; methyl anthranilate; methyl-N-methylanthranilate; Schiff bases of methyl anthranilate with 7 hydroxy-3,7-dimethyloctanal, 2-methyl-3-(4-tert-butylphenyl)propanal or 2,4 dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3 isopropyl-pyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2 ethoxy-5-(1-propenyl)phenol; p-cresyl phenylacetate;
the heterocyclic compounds such as e.g. 2,5-dimethyl-4-hydroxy-2H-furan-3-one; 2 ethyl-4-hydroxy-5-methyl-2H-furan-3-one; 3-hydroxy-2-methyl-4H-pyran-4-one; 2 ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis and trans-11-pentadecen-1,15-olide; cis and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

In a preferred embodiment, the at least one non-aroma chemical carrier (ii) is selected from the group consisting of surfactants, oil components, anti-oxidants, deodorant-active agents and solvents.

In the context of the presently claimed invention, a "solvent" serves for the dilution of the compound of formula (I) to be used according to the invention and/or any further component of the composition without having its own aroma.

The amount of solvent(s) is selected depending on the composition.

In yet another preferred embodiment, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

In yet another preferred embodiment, the solvent is present in the composition in an amount of 0.01 wt.% to 99.0 wt.%, more preferably in an amount of 0.05 wt.% to 95.0 wt.%, yet more preferably in an amount of 0.1 wt.% to 80.0 wt.%, most preferably 0.1 wt.% to 70.0 wt.%, particularly in an amount of 0.1 wt.% to 60.0 wt.%, based on the total weight of the composition.

In yet another preferred embodiment of the invention, the composition comprises 0.05 wt.% to 10 wt.%, more preferably 0.1 wt.% to 5 wt.%, yet more preferably 0.2 wt.% to 3 wt.% solvent(s), based on the total weight of the composition. In yet another preferred embodiment of the invention, the composition comprises 20 wt.% to 70 wt.%, more preferably 25 wt.% to 50 wt.% of solvent(s), based on the total weight of the composition.

One embodiment of the invention is directed to a composition comprising the compound of formula (I) and at least one oil component.

In a preferred embodiment, the oil components are present in an amount of 0.1 to 80 wt.%, more preferably 0.5 to 70 wt.%, yet more preferably 1 to 60 wt.%, even more preferably 1 to 50 wt.%, particularly 1 to 40 wt.%, more particularly 5 to 25 wt.% and specifically 5 to 15 wt.%, based on the total weight of the composition.

The oil components may be selected, for example, from Guerbet alcohols based on fatty alcohols containing 6 to 18, preferably 8 to 10, carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C18-C38 alkyl-hydroxycarboxylic acids with linear or branched C6-C22 fatty alcohols, more especially dioctyl malate, esters of linear and/or branched fatty acids with polyhydric alcohols (for example propylene glycol, dimer dial or trimer triol), triglycerides based on C6-C10 fatty acids, liquid mono-, di- and triglyceride mixtures based on C6-C18 fatty acids, esters of C6-C22 fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C6-C22 fatty alcohol carbonates such as, for example, dicaprylyl carbonate (Cetiol® CC), Guerbet carbonates based on fatty alcohols containing 6 to 18, preferably 8 to 10, carbon atoms, esters of benzoic acid with linear and/or branched C6 to C22 alcohols (for example Finsolv® TN), linear or branched, symmetrical or nonsymmetrical dialkyl ethers containing 6 to 22 carbon atoms per alkyl group such as, for example, dicaprylyl ether (Cetiol® OE), ring opening products of epoxidized fatty acid esters with polyols and hydrocarbons or mixtures thereof.

It is to be understood that anti-oxidants are able to inhibit or prevent the undesired changes in the compositions to be protected caused by oxygen effects and other oxidative processes. The effect of the anti-oxidants consists in most cases in them acting as free-radical scavengers for the free radicals which arise during autoxidation.

In a preferred embodiment, the anti-oxidant is selected from the group consisting of
- amino acids (for example glycine, alanine, arginine, serine, threonine, histidine, tyrosine, tryptophan) and derivatives thereof,
- imidazoles (e.g. urocanic acid) and derivatives thereof,
- peptides, such as D,L-carnosine, D-carnosine, L-carnosine (=*β*-Alanyl-L-histidin) and derivatives thereof
- carotenoids, carotenes (e.g. alpha-carotene, beta-carotene, lycopene, lutein) or derivatives thereof,
- chlorogenic acid and derivatives thereof,
- lipoic acid and derivatives thereof (for example dihydrolipoic acid),
- auro-thioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, gamma-linoleyl, cholesteryl and glyceryl esters thereof) and salts thereof,
- dilauryl thiodipropionate, distearyl thiodipropionate, thiodipropionic acid and derivatives thereof (esters, ethers, peptides, lipids, nucleotides, nucleosides and salts),
- sulfoximine compounds (for example buthionine sulfoximines, homocysteine sulfoximine, buthionine sulfones, penta-, hexa-, heptathionine sulfoximine)
- (metal) chelating agents (e.g. alpha-hydroxy fatty acids, palmitic acid, phytic acid, lactoferrin),
- alpha-hydroxy acids (for example citric acid, lactic acid, malic acid),
- humic acid, bile acid, bile extracts, bilirubin, biliverdin, boldin (= alkaloid from the plant Peumus boldus, boldo extract,
- EDTA, EGTA and derivatives thereof,
- unsaturated fatty acids and derivatives thereof (e.g. gamma-linolenic acid, linoleic acid, oleic acid),
- folic acid and derivatives thereof,
- ubiquinone and ubiquinol and derivatives thereof,
- vitamin C and derivatives (for example ascorbyl palmitate, Mg ascorbyl phosphate, ascorbyl acetate),
- tocopherols and derivatives (for example vitamin E acetate),
- vitamin A and derivatives (for example vitamin A palmitate),
- coniferyl benzoate of gum benzoin, rutic acid and derivatives thereof, alpha-glycosylrutin, ferulic acid, furfurylideneglucitol,
- butylhydroxytoluene (BHT), butylhydroxyanisole (BHA)
- nordihydroguaiacic acid, nordihydroguaiaretic acid, trihydroxybutyrophenone, uric acid and derivatives thereof, mannose and derivatives thereof,
- superoxide dismutase
- zinc and derivatives thereof (for example ZnO, ZnSO4),
- selenium and derivatives thereof (for example selenomethionine) and
- stilbenes and derivatives thereof (e.g. stilbene oxide, trans-stilbene oxide)

In a preferred embodiment, the anti-oxidant is selected from the group consisting of pentaerythrityl, tetra-di-t-butyl-hydroxyhydrocinnamate, nordihydroguaiaretic acid, ferulic acid, resveratrol, propyl gallate, butylhydroxytoluene (BHT), butylhydroxyanisole (BHA), ascorbyl palmitate and tocopherol.

In yet another preferred embodiment, the compositions according to the presently claimed invention can comprise the anti-oxidant in an amount of 0.001 to 25 wt.-%, preferably 0.005 to 10 wt.-%, more preferably 0.01 to 8 wt.-%, yet more preferably 0.025 to 7 wt.-%, even more preferably 0.05 to 5 wt.-%, based on the total weight of the composition.

Deodorizing compositions (deodorants and antiperspirants) counteract, mask or eliminate body odors. Body odors are formed through the action of skin bacteria on apocrine perspiration which results in the formation of unpleasant-smelling degradation products.

One embodiment of the invention is therefore directed to a composition comprising the compound of formula (I) and at least one deodorant-active agent. In a preferred embodiment, the deodorant-active agent is selected from the groups consisting of anti-perspirants, esterase inhibitors and antibacterial agents.

Suitable antiperspirant is selected from the group consisting of salts of aluminum, zirconium or zinc. Examples are aluminum chloride, aluminum chlorohydrate, aluminum dichlorohydrate, aluminum sesquichlorohydrate and complex compounds thereof, for example with 1,2-propylene glycol, aluminum hydroxyallantoinate, aluminum chloride tartrate, aluminum zirconium trichlorohydrate, aluminum zirconium tetrachlorohydrate, aluminum zirconium pentachlorohydrate and complex compounds thereof, for example with amino acids, such as glycine. Aluminum chlorohydrate, aluminum zirconium tetrachlorohydrate, aluminum zirconium pentachlorohydrate and complex compounds thereof are preferably used.

In a preferred embodiment, the anti-perspirant is selected from the group consisting of aluminum chloride, aluminum chlorohydrate, aluminum dichlorohydrate, aluminum sesquichlorohydrate, aluminum hydroxyallantoinate, aluminum chloride tartrate, aluminum zirconium trichlorohydrate, aluminum zirconium tetrachlorohydrate and aluminum zirconium pentachlorohydrate.

Where perspiration is present in the underarm region, extracellular enzymes-esterases, mainly proteases and/or lipases are formed by bacteria and split the esters present in the perspiration, releasing odors in the process. Suitable esterase inhibitors are for example trialkyl citrates, such as trimethyl citrate, tripropyl citrate, triisopropyl citrate, tributyl citrate and, in particular, triethyl citrate. Esterase inhibitors inhibit enzyme activity and thus reduce odor formation. The free acid is probably released by the cleavage of the citric acid ester and reduces the pH value of the skin to such an extent that the enzymes are inactivated by acylation. Other esterase inhibitors are sterol sulfates or phosphates such as, for example, lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester, and zinc glycinate.

In a preferred embodiment, the esterase inhibitor is selected from the group consisting of trimethyl citrate, tripropyl citrate, triisopropyl citrate, tributyl citrate triethyl citrate, lanosterol, cholesterol, campesterol, stigmasterol, sitosterol sulfate, sitosterol phosphate, glutaric acid, glutaric acid monoethyl ester, glutaric acid diethyl ester, adipic acid, adipic acid monoethyl ester, adipic acid diethyl ester, malonic acid, malonic acid diethyl ester, citric acid, malic acid, tartaric acid, tartaric acid diethyl ester and zinc glycinate.

The compositions according to the presently claimed invention comprises the esterase inhibitor in the range of 0.01 to 20 wt.-%, preferably 0.1 to 10 wt.-% and more particularly 0.5 to 5 wt.-%, based on the total weight of the composition.

The term "anti-bacterial agents" as used herein encompasses substances which have bactericidal and/or bacteriostatic properties. Typically these substances act against gram-positive bacteria such as, for example, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides such as, for example, salicylic acid-n-octyl amide or salicylic acid-n-decyl amide

In a preferred embodiment, the antibacterial agent is selected from the group consisting of chitosan, phenoxyethanol, 5-chloro-2-(2,4-dichlorophenoxy)-phenol, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides.

The composition according to the presently claimed invention comprises the antibacterial agent in the range of 0.01 to 5 wt.% and preferably 0.1 to 2 wt.-%, based on the total weight of the composition.

In a preferred embodiment, the composition preferably comprises a surfactant. Due to the characteristic fragrance property of the compound of formula (I) and its substantivity, tenacity as well as stability, it can especially be used to provide an odor, preferably a fragrance impression or aroma impression to surfactant-containing compositions such as, for example, cleaners (in particular laundry care products and all-purpose cleaners). It can preferably be used to impart a long-lasting a flowery and/or a marine and/or a green and/or a sweet note and/or a rubbery note and/or a nutty note and/or a woody note and/or a dusty note and/or a rooty note and/or a lemon note odiferous impression to a surfactant comprising composition.

In a preferred embodiment, the surfactant is selected from the group consisting of anionic, non-ionic, cationic, amphoteric and zwitterionic surfactants. In yet another preferred embodiment, the surfactant is an anionic surfactant.

The compositions according to the presently claimed invention can thus preferably comprise at least one surfactant. The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in an amount of 0 to 40 wt.%, preferably 0 to 20 wt.%, more preferably 0.1 to 15 wt.%, and particularly 0.1 to 10 wt.%, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolysates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one COO(-) or SO3(-) group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C8 to C18 alkyl or acyl group, contain at least one free amino group and at least one -COOH or -SO₃H group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C12-C18 alkyl or acyl groups and sulfosuccinates and acyl glutamates in the form of their alkali metal or ammonium salts.
Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart® series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolysates.

One embodiment of the presently claimed invention is directed to a composition which is selected from the group consisting of perfume compositions, body care compositions, hygiene articles, cleaning compositions, textile detergent compositions, compositions for scent dispensers, foods, food supplements, pharmaceutical compositions and crop protection compositions.

Said composition is preferably an aroma chemical composition, more preferably a fragrance composition.

Suitable compositions are for example perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions and crop protection compositions.

Perfume compositions can be selected from fine fragrances, air fresheners in liquid form, gel-like form or a form applied to a solid carrier, aerosol sprays, scented cleaners, perfume candles and oils, such as lamp oils or oils for massage.
Examples for fine fragrances are perfume extracts, Eau de Parfums, Eau de Toilettes, Eau de Colognes, Eau de Solide and Extrait Parfum.

Body care compositions include cosmetic compositions and products for oral and dental hygiene, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semi-permanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara, and products for oral and dental hygiene, such as toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, dishwashing compositions both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, soft gels, gel caps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds and other pests (both vertebrate and invertebrate) that damage agricultural crops and forestry.

In a preferred embodiment, the composition further comprises at least one auxiliary agent selected from the group consisting of preservatives, abrasives, anti-acne agents, agents to combat skin aging, anti-cellulite agents, antidandruff agents, anti-inflammatory agents, irritation-preventing agents, irritation-alleviating agents, astringents, sweat-inhibiting agents, antiseptics, anti-statics, binders, buffers, carrier materials, chelating agents, cell stimulants, care agents, hair removal agents, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, waxes, fats, phospholipids, saturated fatty acids, mono- or polyunsaturated fatty acids, α-hydroxy acids, polyhydroxy fatty acids, liquefiers, dyes, color-protection agents, pigments, anti-corrosives, polyols, electrolytes and silicone derivatives.

### Compound

One embodiment of the presently claimed invention is directed to a compound of formula (I'), 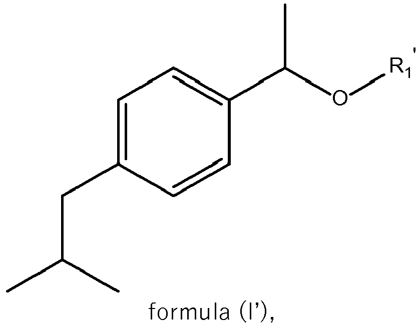 wherein
R₁' is selected from the group consisting of linear or branched -CH₂CH₂CH₃, -CH(CH₃)(C₂H₅), -CH₂CH(CH₃)₂ and -CH₂CH₂CH₂CH₃;
or its salt or stereoisomer thereof.

In a preferred embodiment, R₁' is -CH₂CH₂CH₃. In yet another embodiment, R₁' is-CH(CH₃)(C₂H₅). In yet another preferred embodiment, R₁' is -CH₂CH(CH₃)₂. In yet another embodiment, R₁' is -CH₂CH₂CH₂CH_{3.}

### Embodiments

In the following, there is provided a list of embodiments to further illustrate the present disclosure without intending to limit the disclosure to the specific embodiments listed below.
1. Use of a compound of formula (I), 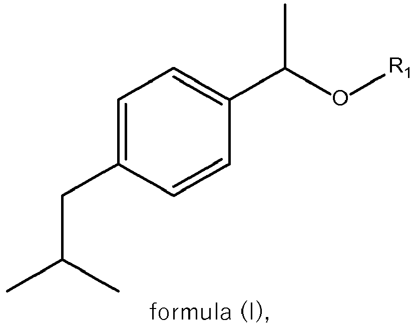 wherein
   R₁ is selected from the group consisting of linear or branched, unsubstituted -C₁-C₅ alkyl and unsubstituted -C₃-C₅ cycloalkyl;
   or its salt or stereoisomer thereof,
   to impart an aroma impression to a composition.
2. Method of imparting an aroma impression to a composition comprising
   at least the step of adding a compound of formula (I), 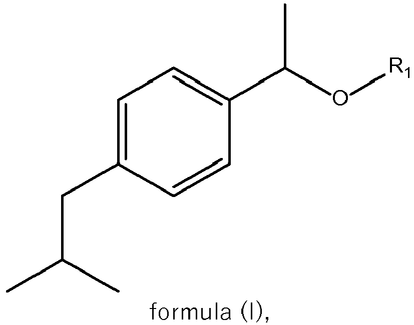 wherein
   R₁ is selected from the group consisting of linear or branched, unsubstituted -C₁-C₅ alkyl and unsubstituted -C₃-C₅ cycloalkyl;
   or its salt or stereoisomer thereof,
   to a composition.
3. The use or method according to any of the preceding embodiments, wherein R₁ is selected from the group consisting of -CH₃, -CH₂CH₃, -CH₂CH₂CH₃, -CH(CH₃)₂,-CH(CH₃)(C₂H₅), -CH₂CH(CH₃)₂, -CH₂CH₂CH₂CH₃ and cyclopropyl.
4. The use or method according to any of the preceding embodiments, wherein the composition selected from perfume compositions, body care compositions, hygiene articles, cleaning compositions, textile detergent compositions, compositions for scent dispensers, foods, food supplements, pharmaceutical compositions and crop protection compositions.
5.The use or method according to any of the preceding embodiments, wherein the impression is selected from a group consisting of a flowery note, a marine note, a sweet note, a rubbery note, a nutty note, a woody note, a dusty note, a rooty note, a lemon note and a green note.
6. The use or method according to any of the preceding embodiments, wherein the compound of formula (I) or its salts or stereoisomer is present in an amount in the range of ≥ 0.01 wt.% to ≤ 70.0 wt.%, based on the total weight of the composition.
7. A composition comprising a at least one compound of the formula (I), 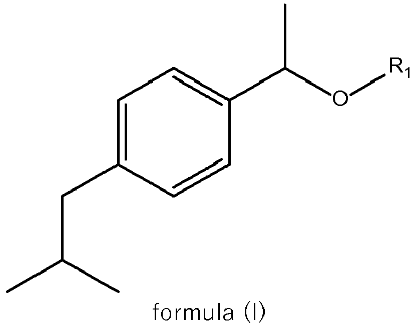 wherein
   R₁ is selected from the group consisting of linear or branched, unsubstituted -C₁-C₅ alkyl and unsubstituted -C₃-C₅ cycloalkyl;
   or its salt or stereoisomer thereof and
   (i) at least one aroma chemical, or
   (ii) at least one non-aroma chemical carrier, or
   (iii) both of (i) and (ii).
8. The composition according to embodiment 7, wherein R₁ is selected from the group consisting of -CH₃, -CH₂CH₃, -CH₂CH₂CH₃, -CH(CH₃)₂, -CH(CH₃)(C₂H₅), -CH₂CH(CH₃)₂, -CH₂CH₂CH₂CH₃, cyclopropyl and cyclobutyl.
9. The composition according to embodiment 7 or 8, wherein the at least one aroma chemical (i) is selected from the group consisting of geranyl acetate, alpha-hexylcinnamaldehyde, 2-phenoxyethyl isobutyrate, dihydromyrcenol, methyl dihydrojasmonate , 4,6,6,7,8,8-hexamethyl-1,3,4,6,7,8-hexahydrocyclopenta[g]benzopyran, tetrahydrolinalool, ethyllinalool, benzyl salicylate, 2-methyl-3-(4-tert-butylphenyl)propanal, cinnamyl alcohol, 4,7-methano-3a,4,5,6,7,7a-hexahydro-5-indenyl acetate and/or 4,7-methano-3a,4,5,6,7,7a-hexahydro-6-indenyl acetate, citronellol, citronellyl acetate, tetrahydrogeraniol, vanillin, linalyl acetate, styrolyl acetate, octahydro-2,3,8,8-tetramethyl-2-acetonaphthone and/or 2-acetyl-1,2,3,4,6,7,8-octahydro-2,3,8,8-tetramethylnaphthalene, hexyl salicylate, 4-tert-butylcyclohexyl acetate, 2-tert-butylcyclohexyl acetate, alpha-ionone, n-alpha-methylionone, alpha-isomethylionone, coumarin, terpinyl acetate, 2-phenylethyl alcohol, 4-(4-hydroxy-4-methylpentyl)-3-cyclohexene-carboxaldehyde, alpha-amylcinnamaldehyde, ethylene brassylate, (E)- and/or (Z)-3-methylcyclopentadec-5-enone, 15-pentadec-11-enolide and/or 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3-(3-isopropylphenyl)butanal, 2-methyl-3-(3,4-methylenedioxyphenyl)propanal, 3-(4-ethylphenyl)-2,2-dimethylpropanal, 7-methyl-2H-1,5-benzodioxepin-3(4H)-one, 3,3,5-trimethylcyclohexyl acetate, 2,5,5-trimethyl-1,2,3,4,4a,5,6,7-octahydronaphthalen-2-ol, 3-(4-*tert*-butylphenyl)-propanal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate and 3-[5,5,6-trimethylbicyclo[2.2.1]hept-2-yl]cyclohexan-1-ol.
10. The composition according to the embodiment embodiment 7 or 8, wherein the at least one aroma chemical (i) is selected from the group consisting of methyl benzoate, benzyl acetate, geranyl acetate, 2-isobutyl-4-methyltetrahydro-2H-pyran-4-ol, linalool, 2-isobutyl-4-methyltetrahydro-2H-pyran-4-ol and methyl benzoate.
11. The composition according to the embodiment embodiment 7 or 8, wherein the at least one aroma chemical (i) is selected from the group consisting of ethylvanillin, vanillin, 2,5-dimethyl-4-hydroxy-2H-furan-3-one (furaneol) or 3-hydroxy-2-methyl-4H-pyran-4-one (maltol).
12. The composition according to any one of the embodiments 8 to 11, wherein the at least one non-aroma chemical carrier (ii) is selected from the group consisting of surfactants, oil components, anti-oxidants, deodorant-active agents and solvents.
13. The composition according to the embodiment 12, wherein the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.
14. The composition according to embodiment 12 or 13, wherein the solvent is present in the composition in an amount of 0.01 wt.% to 99.0 wt.% based on the total weight of the composition.
15. The composition according to the embodiment 12, wherein the at least one deodorant-active agent is selected from the groups consisting of anti-perspirants, esterase inhibitors and antibacterial agents.
16. The composition according to the embodiment 15, wherein the anti-perspirant is selected from selected from the group consisting of aluminum chloride, aluminum chlorohydrate, aluminum dichlorohydrate, aluminum sesquichlorohydrate, aluminum hydroxyallantoinate, aluminum chloride tartrate, aluminum zirconium trichlorohydrate, aluminum zirconium tetrachlorohydrate and aluminum zirconium pentachlorohydrate.
17. The composition according to the embodiment 15, wherein the esterase inhibitor is selected from the group consisting of trimethyl citrate, tripropyl citrate, triisopropyl citrate, tributyl citrate triethyl citrate, lanosterol, cholesterol, campesterol, stigmasterol, sitosterol sulfate, sitosterol phosphate, glutaric acid, glutaric acid monoethyl ester, glutaric acid diethyl ester, adipic acid, adipic acid monoethyl ester, adipic acid diethyl ester, malonic acid, malonic acid diethyl ester, citric acid, malic acid, tartaric acid, tartaric acid diethyl ester and zinc glycinate.
18. The composition according to the embodiment 15, wherein the antibacterial agent is selected from the group consisting of chitosan, phenoxyethanol, 5-Chloro-2-(2,4-dichlorophenoxy)-phenol, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides.
19. The composition according to the embodiment 12, wherein the at least one surfactant is selected from the group consisting of anionic, non-ionic, cationic, amphoteric and zwitterionic surfactants.
20. The composition according to any one of the embodiments 12 or 19, wherein the at least one surfactant is an anionic surfactant.
21. The composition according to the embodiment 12, wherein the at least one anti-oxidant is selected from the group consisting of pentaerythrityl, tetra-di-t-butyl hydroxyhydrocinnamate, nordihydroguaiaretic acid, ferulic acid, resveratrol, propyl gallate, butylhydroxytoluene (BHT), butylhydroxyanisole (BHA), ascorbyl palmitate and tocopherol.
22. The composition according to any of the preceding embodiments 7 to 21, wherein the composition is selected from the group consisting of perfume compositions, body care compositions, hygiene articles, cleaning compositions, textile detergent compositions, compositions for scent dispensers, foods, food supplements, pharmaceutical compositions and crop protection compositions.
23. The composition according to any one of the preceding embodiments 7 to 22, wherein the composition further comprises at least one auxiliary agent selected from the group consisting of preservatives, abrasives, anti-acne agents, agents to combat skin aging, anti-cellulite agents, antidandruff agents, anti-inflammatory agents, irritation-preventing agents, irritation-alleviating agents, astringents, sweat-inhibiting agents, antiseptics, anti-statics, binders, buffers, carrier materials, chelating agents, cell stimulants, care agents, hair removal agents, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, waxes, fats, phospholipids, saturated fatty acids, mono- or polyunsaturated fatty acids, α-hydroxy acids, polyhydroxy fatty acids, liquefiers, dyes, color-protection agents, pigments, anti-corrosives, polyols, electrolytes and silicone derivatives.
24. A compound of formula (I'), 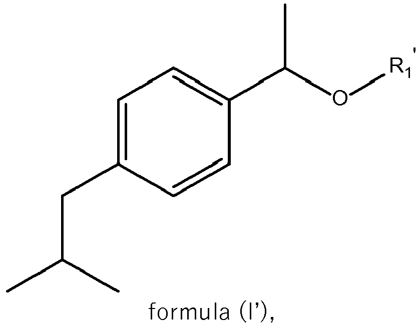 wherein
   R₁' is selected from the group consisting of linear or branched -CH₂CH₂CH₃,-CH(CH₃)(C₂H₅), -CH₂CH(CH₃)₂ and -CH₂CH₂CH₂CH₃;
   or its salt or stereoisomer thereof.

While the presently claimed invention has been described in terms of its specific embodiments, certain modifications and equivalents will be apparent to those skilled in the art and are intended to be included within the scope of the presently claimed invention.

### Examples

The presently claimed invention is illustrated in detail by non-restrictive working examples which follow. More particularly, the test methods specified hereinafter are part of the general disclosure of the application and are not restricted to the specific working examples.

Analytical method: The characterization is done by ¹³C-NMR. The ¹³C-NMR spectra were measured on a Bruker DPX-500 spectrometer

### 1. Preparation of 1-(1-ethoxyethyl)-4-isobutylbenzene

f hydride (60% dispersion in mineral oil, 16.15 g, 0.40 mol, 1.8 eq.) was suspended in dry tetrahydrofuran (THF, 100 mL) at room temperature. A solution of 1-(4-isobutylphenyl)ethanol (40 g, 224 mMol) in dry THF (50 mL) was added dropwise over 30 minutes. After complete addition, stirring was continued for additional 30 minutes before ethyliodide (63.0 g, 0.40 mol, 1.8 eq.) was added dropwise over 45 minutes. After complete addition the temperature was increased to 40 °C and the reaction mixture was stirred for 22 h at 40 °C. Then, the mixture was cooled to room temperature and water (75 mL) was added carefully. The mixture was extracted with methyl tert-butyl ether (MTBE, (2x 75 mL)) and phases were separated. The organic phase was washed with aq. NH₄OH solution (3x 75 mL) and brine (75 mL) and dried over Na₂SO₄. Then, the solvent was removed under reduced pressure (residue 48.8 g). The product was purified by fractional distillation (sump temperature 91-140 °C, pressure 1.5 mBar). Fractions 2-5 were united which contained the desired product in > 95 % purity (in sum 31.2 g, 64 % yield).
¹³C-NMR (125 MHz, CDCl₃): δ = 140.7, 141.4, 129.1, 125.9, 77.6, 63.8, 45.2, 30.3, 24.2, 22.4, 15.4 ppm.

### 2. Preparation of 1-isobutyl-4-(1-methoxyethyl)benzene

The process was carried out analogous to the example 1, by using methyliodide was used for the alkylation. The product was purified by distillation (sump temperature 77-90 °C, pressure 1.5 mBar) and obtained in 97 % purity (65 % yield).
¹³C-NMR (125 MHz, CDCl₃): δ = 141.0, 140.7, 129.2, 126.0, 79.5, 56.4, 45.2, 30.3, 23.9, 22.5 ppm.

### 3. Preparation of 1-isobutyl-4-(1-propoxyethyl)benzene

The process was carried out analogous to example 1, by using a solvent mixture of dry THF and DMSO and 1-iodopropane for the alkylation. An analytical sample was purified by column chromatography (SiO₂, gradient 5-20 % ethylacetate in cyclohexane, 97 % purity).
¹³C-NMR (125 MHz, CDCl₃): δ = 141.5, 140.7, 129.1, 125.9, 77.7, 70.3, 45.2, 30.3, 24.3, 23.2, 22.5, 10.7 ppm.

### 4. Preparation of 1-(1-butoxyethyl)-4-isobutylbenzene

The process was carried out analogous to the example 1, using 1-iodobutane for the alkylation. An analytical sample was purified by column chromatography (SiO₂, gradient 0-20 % ethylacetate in cyclohexane, 95 % purity).
¹³C-NMR (125 MHz, CDCl₃): δ = 141.5, 140.7, 129.1, 125.9, 77.8, 68.4, 45.2, 32.2, 30.3, 24.3, 22.5, 19.5, 14.0 ppm.

**Table 1 - Provides the aroma impression of compound of formula (I)**

| S.No. | compound of formula (I) | aroma impression |
|---|---|---|
| 1. | R₁ is -CH₂CH₃ | watery, marine, mashroom, pungent, green and flowery |
| 2. | R₁ is -CH₃ | watery, sweet, rubbery, green, leafy, nutty |
| 3. | R₁ is -CH₂CH₂CH₃ | nutty, woody, dusty, rubbery, rooty, lemon |
| 4. | R₁ is -CH₂CH₂CH₂CH₃ | Rubbery, waxy |

### Compound A =1-isobutyl-4-(1-methoxyethyl)benzene obtained according to example 2

### Advantageous fragrance compositions

Compound A as described above was formulated in the compositions according to tables 2 and 3. The amounts given in tables 2 and 3 are weight units in grams.

**Table 2: Fragrance compositions 1A and 1B**

| | 1A | 1B |
|---|---|---|
| Lactone C10 gamma (5-hexyloxolan-2-one) | 2 | 2 |
| Bourgeonal (3-(4-tert-butylphenyl)propanal) | 2 | 2 |
| Citronellol | 3 | 3 |
| Aldehyde C-14 (5-heptyloxolan-2-one) | 3 | 3 |
| Allyl heptylate | 4 | 4 |
| Amber core (1-(2-tert-butylcyclohexyl)oxybutan-2-ol) | 4 | 4 |
| Ethyl-2-methyl butyrate | 4 | 4 |
| Geranyl acetate | 5 | 5 |
| Helional (3-(1,3-benzodioxol-5-yl)-2-methylpropanal) | 10 | 10 |
| Manzanate (ethyl 2-methylpentanoate) | 10 | 10 |
| Amberwood (ethoxymethoxycyclododecane) | 10 | 10 |
| Hexyl acetate | 11 | 11 |
| Benzyl salicylate | 12 | 12 |
| Magnolan (2,4-dimethyl-4,4a,5,9b-tetrahydroindeno[1,2-d][1,3]dioxine) | 15 | 15 |
| Verdox (2-tert-butylcyclohexyl) acetate) | 25 | 25 |
| Bergamot oil bergaptene free | 25 | 25 |
| Linalol | 30 | 30 |
| Dipropylene glycol | 45 | 45 |
| Iso E Super (Tetramethyl acetyloctahydronaphthalenes) | 110 | 110 |
| Pyranol (4-methyl-2-(2-methylpropyl)oxan-4-ol) | 170 | 170 |
| Hedione (methyl 3-oxo-2-pentylcyclopentaneacetate) | 200 | 200 |
| Galaxolide 50% IPM (1,3,4,6,7,8-hexahydro-4,6,6,7,8,8-hexamethylcyclopenta(g)-2-benzopyran 50% in isopropyl myristate) | 300 | 300 |
| Compound A | 25 | 50 |
| | 1025 | 1050 |

**Table 3: Fragrance compositions 2A and 2B**

| | 2A | 2B |
|---|---|---|
| Raspberry ketone (4-(4-hydroxyphenyl)butan-2-one) | 4 | 4 |
| Vanitrope (2-ethoxy-5-prop-1-enylphenol) | 6 | 6 |
| Cyclamen aldehyde (at least 90% 2-methyl-3-(p-isopropylphenyl)propionaldehyde; secondary component: 5% 3-(p-cumenyl)-2-methylpropionic acid) | 10 | 10 |
| Bicyclononalactone (3,4,4a,5,6,7,8,8a-octahydrochromen-2-one) | 10 | 10 |
| Aldehyde C-14 (5-heptyloxolan-2-one) | 14 | 14 |
| Ethylvanillin (3-ethoxy-4-hydroxybenzaldehyde) | 16 | 16 |
| Heliotropine (1,3-benzodioxole-5-carbaldehyde) | 20 | 20 |
| Iso E Super (tetramethyl acetyloctahydronaphthalenes) | 20 | 20 |
| Sandela (3-[5,5,6-trimethylbicyclo[2.2.1]hept-2-yl]cyclohexan-1-ol) | 30 | 30 |
| Vanillin isobutyrate ((4-formyl-2-methoxyphenyl) 2-methyl propanoate) | 40 | 40 |
| Aldehyde C-18 (5-pentyloxolan-2-one) | 50 | 50 |
| Benzyl salicylate | 60 | 60 |
| Hexyl cinnamic aldehyde (2-(phenylmethylidene)octanal) | 70 | 70 |
| Hedione (methyl 3-oxo-2-pentylcyclopentaneacetate) | 130 | 130 |
| Pyranol (4-methyl-2-(2-methylpropyl)oxan-4-ol) | 150 | 150 |
| Ethylene brassylate (1,4-dioxacycloheptadecane-5,17-dione) | 170 | 170 |
| Galaxolide 50% IPM (1,3,4,6,7,8-hexahydro-4,6,6,7,8,8-hexamethylcyclopenta(g)-2-benzopyran 50% in isopropyl myristate) | 200 | 200 |
| Compound A | 10 | 50 |
| | 1010 | 1020 |

### 2. Performance test

**Table 1: Deo pump spray PIT 1**

| **Component** | **INCI** | **amount [wt%]** |
|---|---|---|
| EMULGADE® SE-PF | Glyceryl Stearate (and) Ceteareth-20 (and) Ceteareth-12 (and) Cetearyl Alcohol (and) Cetyl Palmitate | 3.9 |
| EUMULGIN® B 2 | Ceteareth-20 | 1.6 |
| CETIOL® OE | Dicaprylyl Ether | 5 |
| CETIOL® PGL | Hexyldecanol (and) Hexyldecyl Laurate | 2 |
| Irgasan DP 300 | Triclosan | 0.25 |
| HYDAGEN® DEO | Triethyl Citrate (and) BHT | 2.5 |
| Eumulgin® HRE 60 | PEG-60 Hydrogenated Castor Oil | 1.5 |
| Fragrance composition 1A | Perfume | 2.0 |
| Preservative | | q.s. |
| Water, deionized | Aqua | ad 100 |
| Viscosity < 100 mPas | | |

Deo pump spray 2 corresponds to deo pump spray 1, where the fragrance composition 1A is replaced by the same amount of the fragrance composition 2A. Deo pump spray 3 corresponds to deo pump spray 1, where the fragrance composition 1A is replaced by the same amount of the fragrance composition 1B. Deo pump spray 4 corresponds to deo pump spray 1, where the fragrance composition 1A is replaced by the same amount of the fragrance composition 2B. Deo pump spray 5 corresponds to deo pump spray 1, where the fragrance composition 1A is replaced by the same amount of the fragrance composition 3. Deo pump spray 6 corresponds to deo pump spray 1, where the fragrance composition 1A is replaced by the same amount of the fragrance composition 4. Deo pump spray 7 corresponds to deo pump spray 1, where the fragrance composition 1A is replaced by the same amount of the fragrance composition 5. Deo pump spray 8 corresponds to deo pump spray 1, where the fragrance composition 1A is replaced by the same amount of the fragrance composition 6.

**Table 2: Deo pump spray 2.1**

| **Component** | **INCI** | **amount [wt%]** |
|---|---|---|
| Eumulgin® HRE 60 | PEG-60 Hydrogenated Castor Oil | 2 |
| Farnesol | Deo ingredient | 0.2 |
| HYDAGEN® DCMF | Chitosan | 0.1 |
| glycolic acid (Fa. Merck) | glycolic acid | 0.04 |
| Fragrance composition 1A | Perfume | 1.0 |
| Water, deionized | | ad 100 |
| pH 4 | | |

Deo pump spray 2.2 corresponds to deo pump spray 2.1, where the fragrance composition 1A is replaced by the same amount of the fragrance composition 2A. Deo pump spray 2.3 corresponds to deo pump spray 2.1, where the fragrance composition 1A is replaced by the same amount of the fragrance composition 1B. Deo pump spray 2.4 corresponds to deo pump spray 2.1, where the fragrance composition 1A is replaced by the same amount of the fragrance composition 2B. Deo pump spray 2.5 corresponds to deo pump spray 2.1, where the fragrance composition 1A is replaced by the same amount of the fragrance composition 3. Deo pump spray 2.6 corresponds to deo pump spray 2.1, where the fragrance composition 1A is replaced by the same amount of the fragrance composition 4. Deo pump spray 2.7 corresponds to deo pump spray 2.1, where the fragrance composition 1A is replaced by the same amount of the fragrance composition 5. Deo pump spray 2.8 corresponds to deo pump spray 2.1, where the fragrance composition 1A is replaced by the same amount of the fragrance composition 6.

**Table 3: Clean Hair Conditioner 1**

| **Component** | **INCI** | **amount [wt.-%]** |
|---|---|---|
| DEHYQUART® L 80 | Dicocoylethyl Hydroxyethylmonium Methosulfate (and) Propylene Glycol | 1.0 |
| Propylene Glycol | solvent | 10.0 |
| Eumulgin® HRE 60 | PEG-60 Hydrogenated Castor Oil | 1.0 |
| LAMESOFT® PO 65 | Coco-Glucoside (and) Glyceryl Oleate | 1.0 |
| GLUADIN® W 40 | Hydrolyzed Wheat Gluten Hydrolyzed Wheat Protein | 0.5 |
| Fragrance composition 1A | Perfume | 1.5 |
| Preservative | | q.s |
| HYDAGEN® HCMF | Chitosan | 10.0 (sol.1%) |
| Water, deionized | | up to 100.0 |
| pH 3.5 - 4.0 | | |

Clean hair conditioner 2 corresponds to clean hair conditioner 1, where the fragrance compo-sition 1A is replaced by the same amount of the fragrance composition 2A. Clean hair condi-tioner 3 corresponds to clean hair conditioner 1, where the fragrance composition 1A is replaced by the same amount of the fragrance composition 1B. Clean hair conditioner 4 corresponds to clean hair conditioner 1, where the fragrance composition 1A is replaced by the same amount of the fragrance composition 2B. Clean hair conditioner 5 corresponds to clean hair conditioner 1, where the fragrance composition 1A is replaced by the same amount of the fragrance composition 3. Clean hair conditioner 6 corresponds to clean hair conditioner 1, where the fragrance composition 1A is replaced by the same amount of the fragrance composition 4. Clean hair conditioner 7 corresponds to clean hair conditioner 1, where the fragrance composition 1A is replaced by the same amount of the fragrance composition 5. Clean hair conditioner 8 corresponds to clean hair conditioner 1, where the fragrance composition 1A is replaced by the same amount of the fragrance composition 6.

**Table 4: Face Wash Gel 1**

| **Component** | **INCI** | **amount [wt.-%]** |
|---|---|---|
| PLANTAPON® 611 L | Sodium Laureth Sulfate (and) Lauryl Glucoside (and) Cocamidopropyl Betaine | 20 |
| PLANTAPON® ACG 35 | Disodium Cocoyl Glutamate | 1 |
| LAMESOFT® PO 65 | Coco-Glucoside (and) Glyceryl Oleate | 2 |
| NaCl | Sodium Chloride | 1.7 |
| PLANTACARE® PS 10 | Sodium Laureth Sulfate (and) Lauryl Polyglucose | 0.3 |
| Fragrance composition 1A | Perfume | 1.0 |
| Preservative | | q.s. |
| Dye | | q.s. |
| Water | Aqua | q.s. |
| pH 6-7 | | |

Face wash gel 2 corresponds to face wash gel 1, where the fragrance composition 1A is replaced by the same amount of the fragrance composition 2A. Face wash gel 3 corresponds to face wash gel 1, where the fragrance composition 1A is replaced by the same amount of the fragrance composition 1B. Face wash gel 4 corresponds to face wash gel 1, where the fragrance composition 1A is replaced by the same amount of the fragrance composition 2B. Face wash gel 5 corresponds to face wash gel 1, where the fragrance composition 1A is replaced by the same amount of the fragrance composition 3. Face wash gel 6 corresponds to face wash gel 1, where the fragrance composition 1A is replaced by the same amount of the fragrance composition 4. Face wash gel 7 corresponds to face wash gel 1, where the fragrance composition 1A is replaced by the same amount of the fragrance composition 5. Face wash gel 8 corresponds to face wash gel 1, where the fragrance composition 1A is replaced by the same amount of the fragrance composition 6.

**Table 5: Foam bath concentrate 1**

| **Component** | **INCI** | **amount [wt%]** |
|---|---|---|
| TEXAPON® K 14 S 70spec. | Sodium Myreth Sulfate | 25 |
| PLANTACARE® 2000 UP | Decyl Glucoside | 20 |
| DEHYTON® K | Cocamidopropyl Betaine | 20 |
| GLUADIN® WP | Hydrolyzed Wheat Gluten Hydrolyzed Wheat Protein | 1 |
| PLANTACARE® PS 10 | Sodium Laureth Sulfate (and) Lauryl Polyglucose | 5 |
| Citric Acid, 50 % | | 0.5 |
| Fragrance composition 1A | perfume | 2.0 |
| Water, deionized | | ad 100 |
| pH 5.5 | | |

Foam bath concentrate 2 corresponds to foam bath concentrate 1, where the fragrance composition 1A is replaced by the same amount of the fragrance composition 2A. Foam bath concentrate 3 corresponds to foam bath concentrate 1, where the fragrance composition 1A is replaced by the same amount of the fragrance composition 1B. Foam bath concentrate 4 corresponds to foam bath concentrate 1, where the fragrance composition 1A is replaced by the same amount of the fragrance composition 2B. Foam bath concentrate 5 corresponds to foam bath concentrate 1, where the fragrance composition 1B is replaced by the same amount of the fragrance composition 3. Foam bath concentrate 6 corresponds to foam bath concentrate 1, where the fragrance composition 1B is replaced by the same amount of the fragrance compo-sition 4. Foam bath concentrate 7 corresponds to foam bath concentrate 1, where the fragrance composition 1B is replaced by the same amount of the fragrance composition 5. Foam bath concentrate 8 corresponds to foam bath concentrate 1, where the fragrance composition 1B is replaced by the same amount of the fragrance composition 6.

**Table 6: Hair Gel 1**

| **Component** | **INCI** | **amount [wt%]** |
|---|---|---|
| HYDAGEN® HCMF | Chitosan | 0.5 |
| Glycolic Acid (Fa. Merck) | Solvent | 0.2 |
| Jaguar HP 105 (2%swelling) | Thickener | 65 |
| Eumulgin® HRE 60 | PEG-60 Hydrogenated Castor Oil | 1.5 |
| Fragrance composition 1A | Perfume | 0.8 |
| Ethanol | Evaporation agent | 7 |
| Water | | ad 100 |
| pH-value 5.8 | | |
| Viscosity (Brookfield RVT 23° C spindle 7, 10 rpm) 20,000 mPas | | |

Hair gel 2 corresponds to hair gel 1, where the fragrance composition 1A is replaced by the same amount of the fragrance composition 2A. Hair gel 3 corresponds to hair gel 1, where the fragrance composition 1A is replaced by the same amount of the fragrance composition 1B. Hair gel 4 corresponds to hair gel 1, where the fragrance composition 1A is replaced by the same amount of the fragrance composition 2B. Hair gel 5 corresponds to hair gel 1, where the fragrance composition 1A is replaced by the same amount of the fragrance composition 3. Hair gel 6 corresponds to hair gel 1, where the fragrance composition 1A is replaced by the same amount of the fragrance composition 4. Hair gel 7 corresponds to hair gel 1, where the fragrance composition 1A is replaced by the same amount of the fragrance composition 5. Hair gel 8 corresponds to hair gel 1, where the fragrance composition 1A is replaced by the same amount of the fragrance composition 6.

**Table 7 Self foaming bodywash 1**

| **Component** | **INCI** | **amount [wt%]** |
|---|---|---|
| TEXAPON® SB 3 KC | Disodium Laureth Sulfosuccinate | 16.5 |
| DEHYTON® K | Cocamidopropyl Betaine | 6.5 |
| PLANTACARE® 818 UP | Coco Glucoside | 7.5 |
| TEXAPON® NSO | Sodium laureth sulfate | 14.2 |
| LAMESOFT® PO 65 | Coco-Glucoside (and) Glyceryl Oleate | 5 |
| Dow Corning 193 | Dimethicole Copolyol | 1 |
| PLANTACARE® PS 10 | Sodium Laureth Sulfate (and) Lauryl Polyglucose | 0.5 |
| Fragrance composition 1A | Perfume | 1.0 |
| Kathon CG | Methylchloroisothiazolinone (and) Methylisothiazolinone | 0.05 |
| Hexylen Glycol | Humectant | 3 |
| UCARE Polymer JR 400 | Polyquaternium-10 | 0.5 |
| Water, deionized | Aqua | ad 100 |
| pH 5.5 | | |
| Viscosity > 100 mPas | | |

Selt-foaming bodywash 2 corresponds to selt-foaming bodywash 1, where the tragrance composition 1A is replaced by the same amount of the fragrance composition 2A. Self-foaming bodywash 3 corresponds to self-foaming bodywash 1, where the fragrance composition 1A is replaced by the same amount of the fragrance composition 1B. Self-foaming bodywash 4 corresponds to self-foaming bodywash 1, where the fragrance composition 1A is replaced by the same amount of the fragrance composition 2B. Self-foaming bodywash 5 corresponds to self-foaming bodywash 1, where the fragrance composition 1A is replaced by the same amount of the fragrance composition 3. Self-foaming bodywash 6 corresponds to self-foaming bodywash 1, where the fragrance composition 1A is replaced by the same amount of the fragrance composition 4. Self-foaming bodywash 7 corresponds to self-foaming bodywash 1, where the fragrance composition 1A is replaced by the same amount of the fragrance composition 5. Self-foaming bodywash 8 corresponds to self-foaming bodywash 1, where the fragrance composition 1A is replaced by the same amount of the fragrance composition 6.

**Table 8: Sprayable sun care emulsion 1**

| **Component** | **INCI** | **amount [wt%]** |
|---|---|---|
| EMULGADE® SE-PF | Glyceryl Stearate (and) Ceteareth-20 (and) Ceteareth-12 (and) Cetearyl Alcohol (and) Cetyl Palmitate | 7.8 |
| EUMULGIN® B 3 | Ceteareth-30 | 4.2 |
| CETIOL® CC | Dicaprylyl Carbonate | 5.0 |
| CETIOL® OE | Dicaprylyl Ether | 5.0 |
| Neo Heliopan® BB | Benzophenone-3 | 4.5 |
| Neo Heliopan® AV | Ethylhexyl Methoxycinnamate | 7.5 |
| Neo Heliopan® 357 | Butyl Methoxydibenzoylmethane | 2.0 |
| Fragrance composition 1A | Perfume | 1.5 |
| Eumulgin® HRE 60 | PEG-60 Hydrogenated Castor Oil | 1.2 |
| Water, de-ionized | | ad 100 |
| pH 5.5 | | |
| Viscosity (RVF spindle 2, 20°C, 50 rpm) < 100 mPas | | |

Sprayable sun care emulsion 2 corresponds to sprayable sun care emulsion 1, where the fragrance composition 1A is replaced by the same amount of the fragrance composition 2A. Spray-able sun care emulsion 3 corresponds to sprayable sun care emulsion 1, where the fragrance composition 1A is replaced by the same amount of the fragrance composition 1B. Sprayable sun care emulsion 4 corresponds to sprayable sun care emulsion 1, where the fragrance composition 1A is replaced by the same amount of the fragrance composition 2B. Sprayable sun care emulsion 5 corresponds to sprayable sun care emulsion 1, where the fragrance composition 1A is replaced by the same amount of the fragrance composition 3. Sprayable sun care emulsion 6 corresponds to sprayable sun care emulsion 1, where the fragrance composition 1A is replaced by the same amount of the fragrance composition 4. Sprayable sun care emulsion 7 corresponds to sprayable sun care emulsion 1, where the fragrance composition 1A is replaced by the same amount of the fragrance composition 5. Sprayable sun care emulsion 8 corresponds to sprayable sun care emulsion 1, where the fragrance composition 1A is replaced by the same amount of the fragrance composition 6.

**Table 9: Sprayable sun protection emulsion 1**

| **Component** | **INCI** | **amount [wt%]** |
|---|---|---|
| EMULGADE® SE-PF | Glyceryl Stearate (and) Ceteareth-20 (and) Ceteareth-12 (and) Cetearyl Alcohol (and) Cetyl Palmitate | 9.0 |
| Eumulgin B3 | Ceteareth-33 | 6.0 |
| CETIOL® CC | Dicaprylyl Carbonate | 4.0 |
| Eusolex HMS | Homosalate | 7.0 |
| Parsol MCX | Ethylhexyl Methoxycinnamate | 7.5 |
| Parsol 1789 | Butyl Methoxydibenzoylmethane | 3.0 |
| Neo Heliopan OS | Ethylhexyl Salicylate | 5.0 |
| Preservative | | q.s. |
| **Fragrance** composition 1A | Perfume | 1.0 |
| Eumulgin® HRE 60 | PEG-60 Hydrogenated Castor Oil | 1.2 |
| Water, de-ionized | | ad 100 |
| pH 6.2 | | |

Sprayable sun protection emulsion 2 corresponds to sprayable sun protection emulsion 1, where the fragrance composition 1A is replaced by the same amount of the fragrance composition 2A. Sprayable sun protection emulsion 3 corresponds to sprayable sun protection emulsion 1, where the fragrance composition 1A is replaced by the same amount of the fragrance composition 1B. Sprayable sun protection emulsion 4 corresponds to sprayable sun protection emulsion 1, where the fragrance composition 1A is replaced by the same amount of the fragrance composition 2B. Sprayable sun protection emulsion 5 corresponds to sprayable sun protection emulsion 1, where the fragrance composition 1A is replaced by the same amount of the fragrance composition 3. Sprayable sun protection emulsion 6 corresponds to sprayable sun protection emulsion 1, where the fragrance composition 1A is replaced by the same amount of the fragrance composition 4. Sprayable sun protection emulsion 7 corresponds to sprayable sun protection emulsion 1, where the fragrance composition 1A is replaced by the same amount of the fragrance composition 5. Sprayable sun protection emulsion 8 corresponds to sprayable sun protection emulsion 1, where the fragrance composition 1A is replaced by the same amount of the fragrance composition 6.

**Table 10: Emollient facial gel 1**

| **Component** | **INCI** | **amount [wt%]** |
|---|---|---|
| HISPAGEL® 200 | Glycerin (and) Glyceryl Polyacrylate | 10 |
| CET-OE-Primasponge®BLUE | | 0.5 |
| PLANTACARE® PS 10 | SodiumLaurethSulfate (and) LaurylPolyglucose | 0.6 |
| Fragrance composition 1A | | 1.0 |
| Water | | ad 100 |

Emollient facial gel 2 corresponds to emollient facial gel 1, where the fragrance composition 1A is replaced by the same amount of the fragrance composition 2A. Emollient facial gel 3 corresponds to emollient facial gel 1, where the fragrance composition 1A is replaced by the same amount of the fragrance composition 1B. Emollient facial gel 4 corresponds to emollient facial gel 1, where the fragrance composition 1A is replaced by the same amount of the fragrance composition 2B. Emollient facial gel 5 corresponds to emollient facial gel 1, where the fragrance composition 1A is replaced by the same amount of the fragrance composition 3. Emollient facial gel 6 corresponds to emollient facial gel 1, where the fragrance composition 1A is replaced by the same amount of the fragrance composition 4. Emollient facial gel 7 corresponds to emollient facial gel 1, where the fragrance composition 1A is replaced by the same amount of the fragrance composition 5. Emollient facial gel 8 corresponds to emollient facial gel 1, where the fragrance composition 1A is replaced by the same amount of the fragrance composition 6.

**Table 11: 2-hases oil foam bath 1**

| **Component** | **INCI** | **amount [wt.-%]** |
|---|---|---|
| PLANTACARE® PS 10 | Sodium Laureth Sulfate (and) Lauryl Polyglucose | 10 |
| Paraffin oil | (low viscosity) | 20 |
| TEXAPON® N 70 | Sodium Laureth sulfate | 13 |
| DEHYTON® PK 45 | Cocamidopropyl Betaine | 8 |
| Fragrance composition 1A | Perfume | 2 |
| Glycerin (86%) | | 5 |
| Preservative | | q.s. |
| Water, de-ionized | | ad 100.0 |
| pH 5.5 | | |

2-phases oil foam bath 2 corresponds to 2-phases oil foam bath 1, where the fragrance composition 1A is replaced by the same amount of the fragrance composition 2A. 2-phases oil foam bath 3 corresponds to 2-phases oil foam bath 1, where the fragrance composition 1A is replaced by the same amount of the fragrance composition 1B. 2-phases oil foam bath 4 corresponds to 2-phases oil foam bath 1, where the fragrance composition 1A is replaced by the same amount of the fragrance composition 2B. 2-phases oil foam bath 5 corresponds to 2-phases oil foam bath 1, where the fragrance composition 1A is replaced by the same amount of the fragrance composition 3. 2-phases oil foam bath 6 corresponds to 2-phases oil foam bath 1, where the fragrance composition 1A is replaced by the same amount of the fragrance composition 4. 2-phases oil foam bath 7 corresponds to 2-phases oil foam bath 1, where the fragrance composition 1A is replaced by the same amount of the fragrance composition 5. 2-phases oil foam bath 8 corresponds to 2-phases oil foam bath 1, where the fragrance composition 1A is replaced by the same amount of the fragrance composition 6.

**Table 12: Shampoos 1, 2, 3 and 4**

| **Shampoo** | | | **1** | **2** | **3** | **4** |
|---|---|---|---|---|---|---|
| **Component** | **INCI** | **Concentration [wt%]** | **amount [wt%]** | | | |
| Texapon N70 | Sodium Laureth Sulfate | 70 | 12.9 | 12.9 | 14.3 | 14.3 |
| Dehyton PK45 | Cocamidopropyl Betaine | 44-46 | 5.4 | 5.4 | 5.4 | 5.4 |
| Plantacare 818UP | Coco-Glucoside | 51-53 | 2.0 | 2.0 | --- | --- |
| PLANTACARE® PS 10 | Sodium Laureth Sulfate (and) Lauryl Polyglucose | 33 | 6.0 | 18.0 | 21.0 | 30.0 |
| Cophero11250 C | Tocopheryl Acetate | 100 | 1.0 | --- | **-**-- | --- |
| Cetiol J 600 | Oleyl Erucate | 100 | --- | 0.7 | --- | 1.0 |
| Almond Oil | Prunus Amygdalus Dulcis (Sweet Almond) Oil | 100 | --- | --- | 0.3 | --- |
| Jaguar C162 | Hydroxypropyl Guar Hydroxypropyl-trimonium Chloride | 100 | 0.2 | 0.2 | --- | --- |
| Jaguar Excel | Guar Hydroxypropyl-trimonium Chloride | 100 | --- | --- | 0.25 | 0.25 |
| Arlypon TT | PEG/PPG-120/10 Trimethylolpropane Trioleate (and) Laureth-2 | 40-50 | 1.0 | 2.0 | 1.0 | 2.0 |
| | | 40-50 | | | | |
| Fragrance composition 1A | Fragrance | 100 | 1.5 | 1.5 | 1.5 | 1.5 |
| Na-Benzoate | Sodium Benzoate | 100 | 0.5 | 0.5 | 0.5 | 0.5 |
| Citric Acid | Citric Acid | 50 | q.s. | q.s. | q.s. | q.s. |
| NaCl | Sodium Chloride | 100 | q.s. | q.s. | q.s. | q.s. |
| Water | Water | 100 | 70.7 | 49.0 | 56.95 | 46.25 |
| pH value | | | 5.0 | 4.9 | 5.2 | 5.1 |
| Viscosity [mPas] | | | 5600 | 3100 | 4600 | 2500 |

**Table B13: Shampoos 5, 6, 7 and 8**

| **Shampoo** | | | | **5** | **6** | **7** | **8** |
|---|---|---|---|---|---|---|---|
| **Component** | **INCI** | | **Concentration [wt%]** | **amount [wt%]** | | | |
| Sulfopon 1216 G | Sodium Coco-Sulfate | | 90-95 | --- | --- | 12.8 | --- |
| Texapon ALS Benz | Ammonium Lauryl Sulfate | | 27-28 | --- | --- | --- | 32.0 |
| Plantacare 1200UP | Lauryl Glucoside | | 51-53 | 15.0 | --- | --- | --- |
| Plantacare 2000UP | Decyl Glucoside | | 51-55 | --- | --- | --- | 6.0 |
| Plantacare 818UP | Coco-Glucoside | | 51-53 | --- | 30.0 | 20.4 | --- |
| Plantacare 810UP | Caprylyl/Capryl Glucoside | | 62-65 | 12.0 | --- | --- | --- |
| Jaguar Excel | Guar Hydroxypropyltrimonium Chloride | | 100 | 0.2 | 0.25 | 0.2 | 0.3 |
| Dehyton PK45 | Cocamidopropyl Betaine | | 44-46 | | | | 11.0 |
| Lamesoft PO65 | Coco-Glucoside (and) Glyceryl Oleate water | 15 - 25 | 28.5-34 | 2.0 | 2.0 | 2.0 | 3.0 |
| | | 10-20 | | | | | |
| | | 62 - 67 | | | | | |
| Euperlan Green | Lauryl Glucoside (and) Stearyl Citrate water | 15 - 25 | 38-44 | --- | --- | --- | 2.0 |
| | | 15 - 25 | | | | | |
| | | 55 - 65 | | | | | |
| Gluadin WLM Benz | Hydrolyzed Wheat Protein | | 21-24 | --- | 0.5 | 1.0 | --- |
| PLANTACARE® PS 10 | Sodium Laureth Sulfate (and) Lauryl Polyglucose | | 33 | 6.0 | 15.0 | 7.5 | 10.5 |
| Copherol 1250 C | Tocopheryl Acetate | | 100 | 0.25 | --- | --- | --- |
| Cetiol J 600 | Oleyl Erucate | | 100 | --- | 0.3 | --- | --- |
| Almond Oil | Prunus Amygdalus Dulcis (Sweet Almond) Oil | | 100 | --- | --- | 0.2 | 0.25 |
| Keltrol CG-SFT | Xanthan Gum | | 100 | 1.0 | 1.0 | | |
| Fragrance composition 1A | Fragrance | | 100 | 1.0 | 1.0 | 1.0 | 1.0 |
| Na-Benzoate | Sodium Benzoate | | 100 | 0.5 | 0,5 | 0.5 | 0.5 |
| Citric Acid | Citric Acid | | 50 | q.s. | q.s. | q.s. | q.s. |
| NaCl | Sodium Chloride | | 100 | q.s. | q.s. | q.s. | q.s. |
| Floral water | Floral water | | 100 | --- | --- | --- | 10.0 |
| Water | Water | | 100 | 62.8 | 50.2 | 55.15 | 24.2 |
| pH value | | | | 5.1 | 5.0 | 5.3 | 4.8 |
| Viscosity [mPas] | | | | 6200 | 4600 | 6800 | 5800 |
| Density [g/cm³] | | | | | | | 1.028 |

Shampoo 9 corresponds to shampoo 1, where the fragrance composition 1A is replaced by the same amount of the fragrance composition 2A. Shampoo 10 corresponds to shampoo 2, where the fragrance composition 1A is replaced by the same amount of the fragrance composition 2A. Shampoo 11 corresponds to shampoo 3, where the fragrance composition 1A is replaced by the same amount of the fragrance composition 2A. Shampoo 12 corresponds to shampoo 4, where the fragrance composition 1A is replaced by the same amount of the fragrance composition 2A. Shampoo 13 corresponds to shampoo 5, where the fragrance composition 1A is replaced by the same amount of the fragrance composition 2A. Shampoo 14 corresponds to shampoo 6, where the fragrance composition 1A is replaced by the same amount of the fragrance composition 2A. Shampoo 15 corresponds to shampoo 7, where the fragrance composition 1A is replaced by the same amount of the fragrance composition 2A. Shampoo 16 corresponds to shampoo 8, where the fragrance composition 1A is replaced by the same amount of the fragrance composition 2A. Shampoo 17 corresponds to shampoo 1, where the fragrance composition 1A is replaced by the same amount of the fragrance composition 1B. Shampoo 18 corresponds to shampoo 2, where the fragrance composition 1A is replaced by the same amount of the fragrance composition 1B. Shampoo 19 corresponds to shampoo 3, where the fragrance composition 1A is replaced by the same amount of the fragrance composition 1B. Shampoo 20 corresponds to shampoo 4, where the fragrance composition 1A is replaced by the same amount of the fragrance composition 1B. Shampoo 21 corresponds to shampoo 5, where the fragrance composition 1A is replaced by the same amount of the fragrance composition 1B. Shampoo 22 corresponds to shampoo 6, where the fragrance composition 1A is replaced by the same amount of the fragrance composition 1B. Shampoo 23 corresponds to shampoo 7, where the fragrance composition 1A is replaced by the same amount of the fragrance composition 1B. Shampoo 24 corresponds to shampoo 8, where the fragrance composition 1A is replaced by the same amount of the fragrance composition 1B. Shampoo 25 corresponds to shampoo 1, where the fragrance composition 1A is replaced by the same amount of the fragrance composition 2B. Shampoo 26 corresponds to shampoo 2, where the fragrance composition 1A is replaced by the same amount of the fragrance composition 2B. Shampoo 27 corresponds to shampoo 3, where the fragrance composition 1A is replaced by the same amount of the fragrance composition 2B. Shampoo 28 corresponds to shampoo 4, where the fragrance composition 1A is replaced by the same amount of the fragrance composition 2B. Shampoo 29 corresponds to shampoo 5, where the fragrance composition 1A is replaced by the same amount of the fragrance composition 2B. Shampoo 30 corresponds to shampoo 6, where the fragrance composition 1A is replaced by the same amount of the fragrance composition 2B. Shampoo 31 corresponds to shampoo 7, where the fragrance composition 1A is replaced by the same amount of the fragrance composition 2B. Shampoo 32 corresponds to shampoo 8, where the fragrance composition 1A is replaced by the same amount of the fragrance composition 2B. Shampoo 33 corresponds to shampoo 1, where the fragrance composition 1A is replaced by the same amount of the fragrance composition 3. Shampoo 34 corresponds to shampoo 2, where the fragrance composition 1A is replaced by the same amount of the fragrance composition 3. Shampoo 35 corresponds to shampoo 3, where the fragrance composition 1A is replaced by the same amount of the fragrance composition 3. Shampoo 36 corresponds to shampoo 4, where the fragrance composition 1A is replaced by the same amount of the fragrance composition 3. Shampoo 37 corresponds to shampoo 5, where the fragrance composition 1A is replaced by the same amount of the fragrance composition 3. Shampoo 38 corresponds to shampoo 6, where the fragrance composition 1A is replaced by the same amount of the fragrance composition 3. Shampoo 39 corresponds to shampoo 7, where the fragrance composition 1A is replaced by the same amount of the fragrance composition 3. Shampoo 40 corresponds to shampoo 8, where the fragrance composition 1A is replaced by the same amount of the fragrance composition 3. Shampoo 41 corresponds to shampoo 1, where the fragrance composition 1A is replaced by the same amount of the fragrance composition 4. Shampoo 42 corresponds to shampoo 2, where the fragrance composition 1A is replaced by the same amount of the fragrance composition 4. Shampoo 43 corresponds to shampoo 3, where the fragrance composition 1A is replaced by the same amount of the fragrance composition 4. Shampoo 44 corresponds to shampoo 4, where the fragrance composition 1A is replaced by the same amount of the fragrance composition 4. Shampoo 45 corresponds to shampoo 5, where the fragrance composition 1A is replaced by the same amount of the fragrance composition 4. Shampoo 46 corresponds to shampoo 6, where the fragrance composition 1A is replaced by the same amount of the fragrance composition 4. Shampoo 47 corresponds to shampoo 7, where the fragrance composition 1A is replaced by the same amount of the fragrance composition 4. Shampoo 48 corresponds to shampoo 8, where the fragrance composition 1A is replaced by the same amount of the fragrance composition 4. Shampoo 49 corresponds to shampoo 1, where the fragrance composition 1A is replaced by the same amount of the fragrance composition 5. Shampoo 50 corresponds to shampoo 2, where the fragrance composition 1A is replaced by the same amount of the fragrance composition 5. Shampoo 51 corresponds to shampoo 3, where the fragrance composition 1A is replaced by the same amount of the fragrance composition 5. Shampoo 52 corresponds to shampoo 4, where the fragrance composition 1A is replaced by the same amount of the fragrance composition 5. Shampoo 53 corresponds to shampoo 5, where the fragrance composition 1A is replaced by the same amount of the fragrance composition 5. Shampoo 54 corresponds to shampoo 6, where the fragrance composition 1A is replaced by the same amount of the fragrance composition 5. Shampoo 55 corresponds to shampoo 7, where the fragrance composition 1A is replaced by the same amount of the fragrance composition 5. Shampoo 56 corresponds to shampoo 8, where the fragrance composition 1A is replaced by the same amount of the fragrance composition 5. Shampoo 57 corresponds to shampoo 1, where the fragrance composition 1A is replaced by the same amount of the fragrance composition 6. Shampoo 58 corresponds to shampoo 2, where the fragrance composition 1A is replaced by the same amount of the fragrance composition 6. Shampoo 59 corresponds to shampoo 3, where the fragrance composition 1A is replaced by the same amount of the fragrance composition 6. Shampoo 60 corresponds to shampoo 4, where the fragrance composition 1A is replaced by the same amount of the fragrance composition 6. Shampoo 61 corresponds to shampoo 5, where the fragrance composition 1A is replaced by the same amount of the fragrance composition 6. Shampoo 62 corresponds to shampoo 6, where the fragrance composition 1A is replaced by the same amount of the fragrance composition 6. Shampoo 63 corresponds to shampoo 7, where the fragrance composition 1A is replaced by the same amount of the fragrance composition 6. Shampoo 64 corresponds to shampoo 8, where the fragrance composition 1A is replaced by the same amount of the fragrance composition 6.

**Table 14: Shower bath 1 2 3 and 4**

| **Shower bath** | | | | **1** | **2** | **3** | **4** |
|---|---|---|---|---|---|---|---|
| **Component** | **INCI** | | **Concentration [wt%]** | **amount [wt%]** | | | |
| Plantapon SF | Sodium Cocoamphoacetate (and) | 10-20 | | 45.0 | 50.0 | 48.0 | 44.0 |
| | Glycerin (and) | 5-15 | 42-52 | | | | |
| | Lauryl Glucoside (and) | 5-15 | | | | | |
| | Sodium Cocoyl Glutamate (and) | 5 | | | | | |
| | Sodium Lauryl Glucose Carboxylate | 5 | | | | | |
| | water | 48-58 | | | | | |
| Lamesoft PO65 | Coco-Glucoside (and) | 15-25 | 28.5-34 | 2.0 | 2.0 | 2.0 | 3.0 |
| | Glyceryl Oleate | 10-20 | | | | | |
| | water | 62-67 | | | | | |
| Euperlan Green | Lauryl Glucoside (and) | 15-25 | | | | | 2.0 |
| | Stearyl Citrate | 15-25 | 38-44 | --- | --- | --- | |
| | water | 55-65 | | | | | |
| Gluadin WLM Benz | Hydrolyzed Wheat Protein | | 21-24 | --- | --- | 1.0 | 0.5 |
| Keltrol CG-SFT | Xanthan Gum | | 100 | 1.0 | 0.5 | --- | --- |
| PLANTACARE ® PS 10 | Sodium Laureth Sulfate (and) Lauryl Polyglucose | | 33 | 4.5 | 6.0 | 10.5 | 7.5 |
| Copherol1250 | Tocopheryl Acetate | | 100 | 0.1 | --- | --- | --- |
| Cetiol J 600 | Oleyl Erucate | | 100 | --- | 0.2 | --- | --- |
| Almond Oil | Prunus Amygdalus Dulcis (Sweet Almond) Oil | | 100 | --- | --- | 0.15 | 0.1 |
| Fragrance composition 1A | Fragrance | | 100 | 1.5 | 1.5 | 1.5 | 1.5 |
| Na-Benzoate | Sodium Benzoate | | 100 | 0.5 | 0.5 | 0.5 | 0.5 |
| Citric Acid | Citric Acid | | 50 | q.s. | q.s. | q.s. | q.s. |
| Water | Water | | 100 | 46.65 | 40,55 | 37.6 | 42.15 |
| pH value | | | | 5.1 | 4.7 | 4.9 | 4.9 |
| Viscosity [mPas] | | | | 4200 | 4600 | 2900 | 2800 |
| Density [g/cm³] | | | | | | | 1.039 |

**Table 15: Shower bath 5, 6, 7 and 8**

| **Shower bath** | | | **5** | **6** | **7** | **8** |
|---|---|---|---|---|---|---|
| **Component** | **INCI** | **Concentration [wt%]** | **amount [wt%]** | | | |
| Texapon N70 | Sodium Laureth Sulfate | 70 | 12.9 | 12.9 | 14.3 | 14.3 |
| Dehyton PK45 | Cocamidopropyl Betaine | 44-46 | 5.4 | 5.4 | 5.4 | 5.4 |
| Plantacare 818UP | Coco-Glucoside | 51-53 | 2.0 | 2.0 | --- | --- |
| PLANTACARE® PS 10 | Sodium Laureth Sulfate (and) Lauryl Polyglucose | 33 | 9.0 | 9.0 | 10.5 | 10.5 |
| DC 245 | Cyclopentasiloxane | 100 | 1.0 | --- | 1.1 | --- |
| Synfluid PAO2 | Hydrogenated Didecene | 100 | --- | 0.8 | --- | 1.0 |
| Jaguar C162 | Hydroxypropyl Guar Hydroxypropyltrimonium Chloride | 100 | 0.2 | 0.2 | --- | --- |
| AquaCAT CG518 | Guar Hydroxypropyltrimonium Chloride | 10 | --- | --- | 2.5 | 2.5 |
| Arlypon TT | PEG/PPG-120/10 Trimethylolpropane Trioleate (and) Laureth-2 | 40-50 | 1.0 | 1.5 | 1.0 | 1.5 |
| | | 40-50 | | | | |
| Fragrance composition 1A | Fragrance | 100 | 1.0 | 1.0 | 1.0 | 1.0 |
| Na-Benzoate | Sodium Benzoate | 100 | 0.5 | 0.5 | 0.5 | 0.5 |
| Citric Acid | Citric Acid | 50 | q.s. | q.s. | q.s. | q.s. |
| NaCl | Sodium Chloride | 100 | q.s. | q.s. | q.s. | q.s. |
| Water | Water | 100 | 67.7 | 67.4 | 64.4 | 64.0 |
| pH value | | | 5.0 | 4.9 | 5.2 | 5.1 |
| Viscosity [mPas] | | | 6600 | 8100 | 6900 | 9300 |

Shower bath 9 corresponds to shower bath 1, where the fragrance composition 1A is replaced by the same amount of the fragrance composition 2A. Shower bath 10 corresponds to shower bath 2, where the fragrance composition 1A is replaced by the same amount of the fragrance composition 2A. Shower bath 11 corresponds to shower bath 3, where the fragrance composition 1A is replaced by the same amount of the fragrance composition 2A. Shower bath 12 corresponds to shower bath 4, where the fragrance composition 1A is replaced by the same amount of the fragrance composition 2A. Shower bath 13 corresponds to shower bath 5, where the fragrance composition 1A is replaced by the same amount of the fragrance composition 2A. Shower bath 14 corresponds to shower bath 6, where the fragrance composition 1A is replaced by the same amount of the fragrance composition 2A. Shower bath 15 corresponds to shower bath 7, where the fragrance composition 1A is replaced by the same amount of the fragrance composition 2A. Shower bath 16 corresponds to shower bath 8, where the fragrance composition 1A is replaced by the same amount of the fragrance composition 2A. Shower bath 17 corresponds to shower bath 1, where the fragrance composition 1A is replaced by the same amount of the fragrance composition 1B. Shower bath 18 corresponds to shower bath 2, where the fragrance composition 1A is replaced by the same amount of the fragrance composition 1B. Shower bath 19 corresponds to shower bath 3, where the fragrance composition 1A is replaced by the same amount of the fragrance composition 1B. Shower bath 20 corresponds to shower bath 4, where the fragrance composition 1A is replaced by the same amount of the fragrance composition 1B. Shower bath 21 corresponds to shower bath 5, where the fragrance composition 1A is replaced by the same amount of the fragrance composition 1B. Shower bath 22 corresponds to shower bath 6, where the fragrance composition 1A is replaced by the same amount of the fragrance composition 1B. Shower bath 23 corresponds to shower bath 7, where the fragrance composition 1A is replaced by the same amount of the fragrance composition 1B. Shower bath 24 corresponds to shower bath 8, where the fragrance composition 1A is replaced by the same amount of the fragrance composition 1B. Shower bath 25 corresponds to shower bath 1, where the fragrance composition 1A is replaced by the same amount of the fragrance composition 2B. Shower bath 26 corresponds to shower bath 2, where the fragrance composition 1A is replaced by the same amount of the fragrance composition 2B. Shower bath 27 corresponds to shower bath 3, where the fragrance composition 1A is replaced by the same amount of the fragrance composition 2B. Shower bath 28 corresponds to shower bath 4, where the fragrance composition 1A is replaced by the same amount of the fragrance composition 2B. Shower bath 29 corresponds to shower bath 5, where the fragrance composition 1A is replaced by the same amount of the fragrance composition 2B. Shower bath 30 corresponds to shower bath 6, where the fragrance composition 1A is replaced by the same amount of the fragrance composition 2B. Shower bath 31 corresponds to shower bath 7, where the fragrance composition 1A is replaced by the same amount of the fragrance composition 2B. Shower bath 32 corresponds to shower bath 8, where the fragrance composition 1A is replaced by the same amount of the fragrance composition 2B. Shower bath 33 corresponds to shower bath 1, where the fragrance composition 1A is replaced by the same amount of the fragrance composition 3. Shower bath 34 corresponds to shower bath 2, where the fragrance composition 1A is replaced by the same amount of the fragrance composition 3. Shower bath 35 corresponds to shower bath 3, where the fragrance composition 1A is replaced by the same amount of the fragrance composition 3. Shower bath 36 corresponds to shower bath 4, where the fragrance composition 1A is replaced by the same amount of the fragrance composition 3. Shower bath 37 corresponds to shower bath 5, where the fragrance composition 1A is replaced by the same amount of the fragrance composition 3. Shower bath 38 corresponds to shower bath 6, where the fragrance composition 1A is replaced by the same amount of the fragrance composition 3. Shower bath 39 corresponds to shower bath 7, where the fragrance composition 1A is replaced by the same amount of the fragrance composition 3. Shower bath 40 corresponds to shower bath 8, where the fragrance composition 1A is replaced by the same amount of the fragrance composition 3. Shower bath 41 corresponds to shower bath 1, where the fragrance composition 1A is replaced by the same amount of the fragrance composition 4. Shower bath 42 corresponds to shower bath 2, where the fragrance composition 1A is replaced by the same amount of the fragrance composition 4. Shower bath 43 corresponds to shower bath 3, where the fragrance composition 1A is replaced by the same amount of the fragrance composition 4. Shower bath 44 corresponds to shower bath 4, where the fragrance composition 1A is replaced by the same amount of the fragrance composition 4. Shower bath 45 corresponds to shower bath 5, where the fragrance composition 1A is replaced by the same amount of the fragrance composition 4. Shower bath 46 corresponds to shower bath 6, where the fragrance composition 1A is replaced by the same amount of the fragrance composition 4. Shower bath 47 corresponds to shower bath 7, where the fragrance composition 1A is replaced by the same amount of the fragrance composition 4. Shower bath 48 corresponds to shower bath 8, where the fragrance composition 1A is replaced by the same amount of the fragrance composition 4. Shower bath 49 corresponds to shower bath 1, where the fragrance composition 1A is replaced by the same amount of the fragrance composition 5. Shower bath 50 corresponds to shower bath 2, where the fragrance composition 1A is replaced by the same amount of the fragrance composition 5. Shower bath 51 corresponds to shower bath 3, where the fragrance composition 1A is replaced by the same amount of the fragrance composition 5. Shower bath 52 corresponds to shower bath 4, where the fragrance composition 1A is replaced by the same amount of the fragrance composition 5. Shower bath 53 corresponds to shower bath 5, where the fragrance composition 1A is replaced by the same amount of the fragrance composition 5. Shower bath 54 corresponds to shower bath 6, where the fragrance composition 1A is replaced by the same amount of the fragrance composition 5. Shower bath 55 corresponds to shower bath 7, where the fragrance composition 1A is replaced by the same amount of the fragrance composition 5. Shower bath 56 corresponds to shower bath 8, where the fragrance composition 1A is replaced by the same amount of the fragrance composition 5. Shower bath 57 corresponds to shower bath 1, where the fragrance composition 1A is replaced by the same amount of the fragrance composition 6. Shower bath 58 corresponds to shower bath 2, where the fragrance composition 1A is replaced by the same amount of the fragrance composition 6. Shower bath 59 corresponds to shower bath 3, where the fragrance composition 1A is replaced by the same amount of the fragrance composition 6. Shower bath 60 corresponds to shower bath 4, where the fragrance composition 1A is replaced by the same amount of the fragrance composition 6. Shower bath 61 corresponds to shower bath 5, where the fragrance composition 1A is replaced by the same amount of the fragrance composition 6. Shower bath 62 corresponds to shower bath 6, where the fragrance composition 1A is replaced by the same amount of the fragrance composition 6. Shower bath 63 corresponds to shower bath 7, where the fragrance composition 1A is replaced by the same amount of the fragrance composition 6. Shower bath 64 corresponds to shower bath 8, where the fragrance composition 1A is replaced by the same amount of the fragrance composition 6.

**Table 16: Hydro-alcoholic AP/Deo pump spray 1**

| **Component** | **INCI** | **amount [wt%]** |
|---|---|---|
| Ethanol, cosm. | Alcohol | 40.0 |
| water, demin. | Aqua | 37.5 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1.5 |
| Chlorhydrol (50% solut.) | Aluminium Chlorohydrate | 8.0 |
| Fragrance composition 1A | | 1.0 |

Deo pump spray 2 corresponds to the hydro-alcoholic AP/Deo pump spray 1, where the fragrance composition 1A is replaced by the same amount of the fragrance composition 2A. Deo pump spray 3 corresponds to the hydro-alcoholic AP/Deo pump spray 1, where the fragrance composition 1A is replaced by the same amount of the fragrance composition 1B. Deo pump spray 4 corresponds to the hydro-alcoholic AP/Deo pump spray 1, where the fragrance composition 1A is replaced by the same amount of the fragrance composition 2B. Deo pump spray 5 corresponds to the hydro-alcoholic AP/Deo pump spray 1, where the fragrance composition 1A is replaced by the same amount of the fragrance composition 3. Deo pump spray 6 corresponds to the hydro-alcoholic AP/Deo pump spray 1, where the fragrance composition 1A is replaced by the same amount of the fragrance composition 4. Deo pump spray 7 corresponds to the hydro-alcoholic AP/Deo pump spray 1, where the fragrance composition 1A is replaced by the same amount of the fragrance composition 5. Deo pump spray 8 corresponds to the hydro-alcoholic AP/Deo pump spray 1, where the fragrance composition 1A is replaced by the same amount of the fragrance composition 6.

**Table 17: Aerosol 1**

| **Component** | **INCI** | **amount [wt%]** |
|---|---|---|
| ethanol, cosmetic | Alcohol | 80.0 |
| water, demineralized | Aqua | 16.7 |
| propylene glycol | Propylene Glycol | 1.0 |
| Eumulgin® HRE 60 | PEG-60 Hydrogenated Castor Oil | 1.5 |
| alpha-Bisabolol | Bisabolol | 0.05 |
| Triclosan | Triclosan | 0.25 |
| Fragrance composition 1A | | 1.0 |

Aerosol 2 corresponds to aerosol 1, where the fragrance composition 1A is replaced by the same amount of the fragrance composition 2A. Aerosol 3 corresponds to aerosol 1, where the fragrance composition 1A is replaced by the same amount of the fragrance composition 1B. Aerosol 4 corresponds to aerosol 1, where the fragrance composition 1A is replaced by the same amount of the fragrance composition 2B. Aerosol 5 corresponds to aerosol 1, where the fragrance composition 1A is replaced by the same amount of the fragrance composition 3. Aerosol 6 corresponds to aerosol 1, where the fragrance composition 1A is replaced by the same amount of the fragrance composition 4. Aerosol 7 corresponds to aerosol 1, where the fragrance composition 1A is replaced by the same amount of the fragrance composition 5. Aerosol 8 corresponds to aerosol 1, where the fragrance composition 1A is replaced by the same amount of the fragrance composition 6.

**Table 18: Aqueous/alcoholic AP/Deo roll-on 1**

| **Component** | **INCI** | **amount [wt%]** |
|---|---|---|
| ethanol, cosmetic | Alcohol | 40.0 |
| water, demineralized | Aqua | 37.0 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1.5 |
| Chlorhydrol (50% solut.) | Aluminium Chlorohydarte | 8.0 |
| Klucel M | Hydroxypropylcellulose | 0.5 |
| Fragrance composition 1A | | 1.0 |

Aqueous/alcoholic AP/Deo roll-on according to table 18, where the fragrance composition 1A is replaced by the same amount of the fragrance composition 2A. Aqueous/alcoholic AP/Deo roll-on according to table 18, where the fragrance composition 1A is replaced by the same amount of the fragrance composition 1B. Aqueous/alcoholic AP/Deo roll-on according to table 18, where the fragrance composition 1A is replaced by the same amount of the fragrance composition 2B. Aqueous/alcoholic AP/Deo roll-on according to table 18, where the fragrance composition 1A is replaced by the same amount of the fragrance composition 3. Aqueous/alcoholic AP/Deo roll-on according to table 18, where the fragrance composition 1A is replaced by the same amount of the fragrance composition 4. Aqueous/alcoholic AP/Deo roll-on according to table 18, where the fragrance composition 1A is replaced by the same amount of the fragrance composition 5. Aqueous/alcoholic AP/Deo roll-on according to table 18, where the fragrance composition 1A is replaced by the same amount of the fragrance composition 6.

**Table 19: Styling Gel Type "Out of Bed"**

| **Phase** | **Component** | **INCI** | **amount [wt.-%]** | **Function** |
|---|---|---|---|---|
| I | Alcool 96% (Prolabo) | Alcohol 96% | 12.00 | Solvent |
| | Luviskol VA 64 (BASF) | PVP/VA | 4.50 | Styling agent |
| | Polyox WSR-301 | PEG-90M | 0.25 | Styling agent |
| | Methocel E4M Premium EP | Hydroxypropyl Methylcellulose | 0.60 | Thickener |
| | DEHYQUART® L 80 | Dicocoylethyl Hydroxyethylmonium Methosulfate (and) Propylene Glycol | 0.60 | Active agent |
| II. | Eumulgin® HRE 60 | Hardened castor oil with approx. 60 mol EO | 1.00 | Solubilizer |
| | CETIOL® OE | Dicaprylyl Ether | 1.50 | Emollient |
| III. | Water, de-ionized | Aqua | a.d. | |
| IV. | HISPAGEL® 200 | Glycerin (and) Glyceryl Polyacrylate | 36.70 | Thickener |
| V. | Fragrance composition 1A | | 0.5 | |
| pH 6.8, Viscosity (Brookfield RVT 23° C spindle 5, 10 rpm) 220,000 mPas | | | | |

Styling gel according to table 19, where the fragrance composition 1A is replaced by the same amount of the fragrance composition 2A. Styling gel according to table 19, where the fragrance composition 1A is replaced by the same amount of the fragrance composition 1B. Styling gel according to table 19, where the fragrance composition 1A is replaced by the same amount of the fragrance composition 2B. Styling gel according to table 19, where the fragrance composition 1A is replaced by the same amount of the fragrance composition 3. Styling gel according to table 19, where the fragrance composition 1A is replaced by the same amount of the fragrance composition 4. Styling gel according to table 19, where the fragrance composition 1A is replaced by the same amount of the fragrance composition 5. Styling gel according to table 19, where the fragrance composition 1A is replaced by the same amount of the fragrance composition 6.

**Table 20: Shaving Foam**

| **Component** | **INCI** | **amount [wt.-%]** | **Function** |
|---|---|---|---|
| CUTINA® FS 45 | StearicAcid (and) Palmitic Acid | 7.6 | Surfactant |
| PLANTACARE® 1200 UP | Lauryl Glucoside | 6.0 | Surfactant (i n h i bitory effect) |
| MONOMULS® 90-0 18 | Glyceryl Oleate | 1.2 | W/O emulsifier |
| EUMULGIN® O20 S | Oleth-20 | 1.2 | O/W-emulsifier |
| CETIOL® CC | Dicaprylyl Carbonate | 2.0 | Emollient (low viscosity) |
| DEHYQUART® SP | Quaternium-52 | 0.5 | Surfactant |
| TEA (99%) | Triethanolamine | 4.0 | Neutralizer |
| Glycerin | Glycerin | 3.0 | Humectant |
| Propylene Glycol | Propylene Glycol | 3.0 | Humectant |
| Emulgin® HRE 60 | Hardened castor oil with approx. 60 mol EO | 2.0 | Solubilizer |
| D-Panthenol | Panthenol | 0.2 | Care additive |
| Phenonip | Phenoxyethanol (and) Parabens | 0.5 | Preservative |
| Fragrance composition 1A | | 1.0 | Fragrance |
| Distilled or Deionized Water | Aqua | ad 100 | |

Shaving foam according to table 20, where the fragrance composition 1A is replaced by the same amount of the fragrance composition 2A. Shaving foam according to table 20, where the fragrance composition 1A is replaced by the same amount of the fragrance composition 1B. Shaving foam according to table 20, where the fragrance composition 1A is replaced by the same amount of the fragrance composition 2B. Shaving foam according to table 20, where the fragrance composition 1A is replaced by the same amount of the fragrance composition 3. Shaving foam according to table 20, where the fragrance composition 1A is replaced by the same amount of the fragrance composition 4. Shaving foam according to table 20, where the fragrance composition 1A is replaced by the same amount of the fragrance composition 5. Shaving foam according to table 20, where the fragrance composition 1A is replaced by the same amount of the fragrance composition 6.

**Table 21: Sensitive skin Baby shampoo**

| **Ingredients** | | **INCI / Chemistry** | **amount [wt%]** | **Function** |
|---|---|---|---|---|
| PLANTAPON® LGC SORB | | Sodium Lauryl Glucose Carboxylate (and) Lauryl Glucoside | 28.00 | Surfactant |
| ARLYPON® TT | | PEG/PPG-120/10 Trimethylolpropane Trioleate (and) Laureth-2 | 2.00 | Thickener |
| DEHYTON® MC | | Sodium Cocoamphoacetate | 6.00 | Surfactant |
| EUMULGIN® SML 20 | | Polysorbate 20 | 3.00 | Solubilizer |
| LAMESOFT® PO 65 | | Coco-Glucoside (and) Glyceryl Oleate | 2.20 | Lipid layer enhancer |
| MELHYDRAN™ LS 4420 | | Honey Extract | 1.00 | Active ingredient |
| Fragrance composition 1A | | Perfume | 0.5 | Perfume |
| Dermosoft 1388 | | Water (and) Glycerin (and) Sodium | 4.00 | Active ingredient |
| | (Dr. Straetmans) | Levulinate (and) Sodium Anisate | | |
| | Water, demineralized | Aqua | 53.60 | |

Sensitive skin baby shampoo according to table 21, where the fragrance composition 1A is replaced by the same amount of the fragrance composition 2A. Sensitive skin baby shampoo according to table 21, where the fragrance composition 1A is replaced by the same amount of the fragrance composition 1B. Sensitive skin baby shampoo according to table 21, where the fragrance composition 1A is replaced by the same amount of the fragrance composition 2B. Sensitive skin baby shampoo according to table 21, where the fragrance composition 1A is replaced by the same amount of the fragrance composition 3. Sensitive skin baby shampoo according to table 21, where the fragrance composition 1A is replaced by the same amount of the fragrance composition 4. Sensitive skin baby shampoo according to table 21, where the fragrance composition 1A is replaced by the same amount of the fragrance composition 5. Sensitive skin baby shampoo according to table 21, where the fragrance composition 1A is replaced by the same amount of the fragrance composition 6.

**Table 25: Body wash for Sensitive Skin**

| **Ingredients** | | **INCI / Chemistry** | **amount [wt.-%]** | **Function** |
|---|---|---|---|---|
| I. | Water, demin. | Aqua | 70.90 | |
| | TEXAPON® N 70 | Sodium Laureth Sulfate | 13.00 | Surfactant |
| | PLANTACARE® 818 UP | Coco-Glucoside | 3.00 | Surfactant |
| | LAMESOFT® PO 65 | Coco-Glucoside (and) Glyceryl Oleate | 5.00 | Active, skin conditioning |
| | Fragrance composition 1A | Fragrance | 1.0 | Perfume |
| | Sodium Benzoate | Sodium Benzoate | 0.55 | Preservative |
| II. | DEHYQUART® CC7 BZ | Polyquaternium-7 | 2.00 | Active, hair conditioning |
| III. | MELHYDRAN™ LS 4420 | Honey Extract | 1.00 | Active, moisturizing |
| | ARLYPON® TT | PEG/PPG-120/10 Trimethylolpropane Trioleate (and) Laureth-2 | 0.50 | Thickener |
| | DEHYTON® PK 45 | Cocamidopropyl Betaine | 3.80 | Surfactant |
| IV. | Sodium Chloride | Sodium Chloride | 0.10 | Agent, thickening |

Body wash for sensitive skin according to table 25, where the fragrance composition 1A is replaced by the same amount of the fragrance composition 2A. Body wash for sensitive skin according to table 25, where the fragrance composition 1A is replaced by the same amount of the fragrance composition 1B. Body wash for sensitive skin according to table 25, where the fragrance composition 1A is replaced by the same amount of the fragrance composition 2B. Body wash for sensitive skin according to table 25, where the fragrance composition 1A is replaced by the same amount of the fragrance composition 3. Body wash for sensitive skin according to table 25, where the fragrance composition 1A is replaced by the same amount of the fragrance composition 4. Body wash for sensitive skin according to table 25, where the fragrance composition 1A is replaced by the same amount of the fragrance composition 5. Body wash for sensitive skin according to table 25, where the fragrance composition 1A is replaced by the same amount of the fragrance composition 6.

**Table 26: Gloss Enhancing Shampoo for Sensitive Scalp**

| **Ingredients** | | **INCI / Chemistry** | **amount [wt.-%]** | **Function** |
|---|---|---|---|---|
| I. | Water, demin. | Aqua | 71.60 | |
| | Luviquat Ultra Care (BASF) | Polyquaternium-44 | 1.50 | Active, hair conditioning |
| II. | Sodium Benzoate | Sodium Benzoate | 0.55 | Preservative |
| III. | TEXAPON® N 70 | Sodium Laureth Sulfate | 14.30 | Surfactant |
| | DEHYTON® PK 45 | Cocamidopropyl Betaine | 5.40 | Surfactant |
| | MELHYDRAN™ LS 4420 | Honey Extract | 1.00 | Active, moisturizing |
| | Fragrance composition 1A | Fragrance | 1.0 | Perfume |
| IV. | DEHYDOL® LS 2 DEO N | Laureth-2 | 1.00 | Thickener |
| V. | LAMESOFT® CARE | PEG-4 Distearyl Ether (and) Sodium Laureth sulfate (and) Distearyl Ether (and) Dicaprylyl Ether | 3.00 | Active, hair conditioning |
| VI. | Sodium Chloride | Sodium Chloride | 1.50 | Agent, thickening |

Gloss enhancing shampoo for sensitive scalp according to table 26, where the fragrance composition 1A is replaced by the same amount of the fragrance composition 2A. Gloss enhancing shampoo for sensitive scalp according to table 26, where the fragrance composition 1A is replaced by the same amount of the fragrance composition 1B. Gloss enhancing shampoo for sensitive scalp according to table 26, where the fragrance composition 1A is replaced by the same amount of the fragrance composition 2B. Gloss enhancing shampoo for sensitive scalp according to table 26, where the fragrance composition 1A is replaced by the same amount of the fragrance composition 3. Gloss enhancing shampoo for sensitive scalp according to table 26, where the fragrance composition 1A is replaced by the same amount of the fragrance composition 4. Gloss enhancing shampoo for sensitive scalp according to table 26, where the fragrance composition 1A is replaced by the same amount of the fragrance composition 5. Gloss enhancing shampoo for sensitive scalp according to table 26, where the fragrance composition 1A is replaced by the same amount of the fragrance composition 6.

**Table 27: Deo Stick**

| **Ingredients** | | **INCI / Chemistry** | **amount [wt%]** | **Function** |
|---|---|---|---|---|
| I. | LANETTE® 18 DEO | Stearyl Alcohol | 18.50 | Consistency factor |
| | CUTINA® HR POWDER | Hydrogenated Castor Oil | 4.50 | Consistency factor |
| | CUTINA® CP | Cetyl Palmitate | 25.00 | Emollient |
| | CETIOL® MM | Myristyl Myristate | 10.00 | Emollient |
| | CETIOL® RLF | Caprylyl Caprylate/Caprate | 5.00 | Emollient |
| | MYRITOL® 312 | Caprylic/Capric Triglyceride | 29.30 | Emollient |
| | HYDAGEN® C.A.T. | Triethyl Citrate | 5.00 | Active, anti - perspirant |
| II. | COSMEDIA® SILC | Silica | 2.00 | Skin feel modifier |
| | PHYTOSOOTHE™ LS 9766 | Brassica Campestris (Rapeseed) Sterols (and) Cetearyl Alcohol | 0.50 | Active ingredient |
| III. | Fragrance composition 1A | Fragrance | 1.0 | Perfume |

Deo stick according to table 27, where the fragrance composition 1A is replaced by the same amount of the fragrance composition 2A. Deo stick according to table 27, where the fragrance composition 1A is re-placed by the same amount of the fragrance composition 1B. Deo stick according to table 27, where the fragrance composition 1A is replaced by the same amount of the fragrance composition 2B. Deo stick according to table 27, where the fragrance composition 1A is replaced by the same amount of the fragrance composition 3. Deo stick according to table 27, where the fragrance composition 1A is replaced by the same amount of the fragrance composition 4. Deo stick according to table 27, where the fragrance composition 1A is replaced by the same amount of the fragrance composition 5. Deo stick according to table 27, where the fragrance composition 1A is replaced by the same amount of the fragrance composition 6.

**Table 28: Baby Wipe**

| **Ingredients** | | **INCI / Chemistry** | **amount [wt%]** | **Function** |
|---|---|---|---|---|
| I. | Water, demin. | Water | 95.25 | |
| | Sodium Benzoate | Sodium Benzoate | 0.50 | Preservative |
| II. | EMULGADE® CPE | Vegetable Oil (and) Glycerin (and) Lauryl Glucoside (and) Polyglyceryl-2-Dipolyhydroxystearate (and) Glyceryl Oleate | 4.00 | Emulsion base (O/W) |
| | LIPOFRUCTYL™ ARGAN LS 9779 | Argania Spinosa Kernel Oil | 0.10 | Active ingredient |
| | Fragrance composition 1A | Fragrance | 1.0 | Perfume |
| III. | Citric Acid (50 %) | Citric Acid | 0.15 | pH adjusting agent |

Babywipe according to table 28, where the fragrance composition 1A is replaced by the same amount of the fragrance composition 2A. Babywipe according to table 28, where the fragrance composition 1A is replaced by the same amount of the fragrance composition 1B. Babywipe according to table 28, where the fragrance composition 1A is replaced by the same amount of the fragrance composition 2B. Babywipe according to table 28, where the fragrance composition 1A is replaced by the same amount of the fragrance composition 3. Babywipe according to table 28, where the fragrance composition 1A is replaced by the same amount of the fragrance composition 4. Babywipe according to table 28, where the fragrance composition 1A is replaced by the same amount of the fragrance composition 5. Babywipe according to table 28, where the fragrance composition 1A is replaced by the same amount of the fragrance composition 6.

**Table 29: After shave balm**

| | **Ingredients** | **INCI** | **amount [wt%]** | **Function** |
|---|---|---|---|---|
| I | Myritol® 331 | Cocoglycerides | 2.50 | Emollient |
| | Cetiol® Sensoft | Propylheptyl Caprylate | 5.00 | Emollient |
| | Cetiol® C5 | Coco-Caprylate | 2.50 | Emollient |
| | Cosmedia® Triple C | Polyquaternium-37 (and) Dicaprylyl Carbonate (and) Lauryl Glucoside | 1.50 | Cationic Polymer |
| II | Deionized Water | Aqua | 84.80 | |
| | Glycerine | Glycerin | 1.00 | Humectant |
| III | Anasensyl™ LS 9322 | Mannitol (and) Ammonium Glycyrrhizate (and) Caffeine (and) Zinc Gluconate (and) Aesculus Hippocastanum Extract | 1.00 | Soothing Active |
| IV | Fragrance composition 1A | Fragrance | 0.5 | Perfume |
| V | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| VI | NaOH (Aq. Sol. 25%) | Sodium Hydroxide | 0.10 | Neutralizing Agent |

After shave balm according to table 29, where the fragrance composition 1AB is replaced by the same amount of the fragrance composition 2A. After shave balm according to table 29, where the fragrance composition 1A is replaced by the same amount of the fragrance composition 1B. After shave balm according to table 29, where the fragrance composition 1A is replaced by the same amount of the fragrance composition 2B. After shave balm according to table 29, where the fragrance composition 1A is replaced by the same amount of the fragrance composition 3. After shave balm according to table 29, where the fragrance composition 1A is replaced by the same amount of the fragrance composition 4. After shave balm according to table 29, where the fragrance composition 1A is replaced by the same amount of the fragrance composition 5. After shave balm according to table 29, where the fragrance composition 1A is replaced by the same amount of the fragrance composition 6.

**Table 30: Face Gel**

| **Ingredients** | | **INCI** | **amount [wt%]** | **Function** |
|---|---|---|---|---|
| **I** | Deionized Water | Aqua | 71.65 | |
| | Glycerine | Glycerin | 3.00 | Humectant |
| | EDETA B Powder (BASF) | Tetrasodium EDTA | 0.05 | Complexing Agent |
| **II** | Cosmedia® SP | Sodium Polyacrylate | 1.20 | Emulsifying Polymer |
| **III** | Cegesoft® PFO | Passiflora Incarnata Oil | 2.00 | Emollient |
| | Uvinul MC 80 (BASF) | Ethylhexyl Methoxycinnamate | 2.00 | UVB Filter |
| | KF 96, 100 cs (Shin Etsu) | Dimethicone | 2.00 | Emollient |
| **IV** | Cetiol® CC | Dicaprylyl Carbonate | 5.00 | Emollient |
| | Cetiol® C5 | Coco-Caprylate | 4.00 | Emollient |
| | Uvinul A Plus Granular (BASF) | Diethylamino Hydroxybenzoyl Hexyl Benzoate | 0.50 | UVA Filter |
| | Cegesoft® SBE | Butyrospermum Parkii | 1.50 | Emollient |
| | Eumulgin® SG | Sodium Stearoyl Glutamate | 0.10 | O/W Emulsifier |
| **V** | Deionized Water | Aqua | 5.00 | |
| | Skinasensyl™ PW LS 9852 | Mannitol (and) Sodium Citrate (and) Acetyl Tetrapeptide-15 | 0.30 | Soothing Active |
| **VI** | Covi-ox® T70C | Tocopherol | 0.10 | Antioxidant |
| | Fragrance composition 1A | Fragrance | 1.0 | Perfume |
| **VII** | Euxyl PE 9010 (Schülke) | Phenoxyethanol (and) | 1.00 | Preservative |
| | | Ethylhexylglycerin | | |
| | Sensiva SC 50 (Schülke) | Ethylhexylglycerin | 0.50 | Preservative |

Face gel according to table 30, where the fragrance composition 1A is replaced by the same amount of the fragrance composition 2A. Face gel according to table 30, where the fragrance composition 1A is replaced by the same amount of the fragrance composition 1B. Face gel according to table 30, where the fragrance composition 1A is replaced by the same amount of the fragrance composition 2B. Face gel according to table 30, where the fragrance composition 1A is replaced by the same amount of the fragrance composition 3. Face gel according to table 30, where the fragrance composition 1A is replaced by the same amount of the fragrance composition 4. Face gel according to table 30, where the fragrance composition 1A is replaced by the same amount of the fragrance composition 5. Face gel according to table 30, where the fragrance composition 1A is replaced by the same amount of the fragrance composition 6.

**Table 31: Face Day Care Cream**

| **Ingredients** | | **INCI** | **amount [wt%]** | **Function** |
|---|---|---|---|---|
| **I** | Deionized Water | Aqua | 69.55 | |
| | Glycerine | Glycerin | 3.00 | Humectant |
| | EDETA B Powder | Tetrasodium EDTA | 0.05 | Complexing Agent |
| **II** | Cosmedia® SP | Sodium Polyacrylate | 0.80 | Emulsifying Polymer |
| **III** | Emulgade® PL 68/50 | Cetearyl Glucoside (and) Cetearyl Alcohol | 2.00 | Self-Emulsifying Base |
| | Eumulgin® SG | Sodium Stearoyl Glutamate | 0.50 | O/W Emulsifier |
| | Monomuls® 90-0-18 | Glyceryl Oleate | 2.00 | W/O Emulsifier |
| | Cutina® GMS-V | Glyceryl Stearate | 2.00 | Consistency Giving Factor |
| | Cetiol® Sensoft | Propylheptyl Caprylate | 5.00 | Emollient |
| | Myritol® 331 | Cocoglycerides | 3.00 | Emollient |
| | Cegesoft® PFO | Passiflora Incarnata Oil | 3.00 | Emollient |
| | Lipodermol™ LS 8656 | Octyldodecanol (and) Lecithin (and) Arachidyl Propionate (and) Tocopheryl Acetate (and) Retinyl Palmitate (and) Ethyl Linoleate (and) Ethyl Linolenate (and) Ethyl Oleate | 1.00 | Anti-Ageing Active |
| | Generol® R | Brassica Campestris (Rapeseed) Sterols | 1.00 | Anti-inflammatory Active |
| **IV** | Sphingoceryl™ WS LS 9859 | Aqua (and) Glycerin (and) Helianthus Annuus Seed Extract (and) Decyl Glucoside | 1.00 | Moisturizing Active |
| | Deionized Water | Aqua | 4.00 | |
| | Fragrance composi-tion 1A | Fragrance | 1.0 | Perfume |
| **V** | Covi-ox® T70C | Tocopherol | 0.50 | Antioxidant |
| **VI** | Euxyl PE 9010 | Phenoxyethanol (and) Ethylhexylglycerin | 1.00 | Preservative |
| | Sensiva SC 50 | Ethylhexylglycerin | 0.50 | Preservative |

Face day care cream according to table 31, where the fragrance composition 1A is replaced by the same amount of the fragrance composition 2A. Face day care cream according to table 31, where the fragrance composition 1A is replaced by the same amount of the fragrance composition 1B. Face day care cream according to table 31, where the fragrance composition 1A is replaced by the same amount of the fragrance composition 2B. Face day care cream according to table 31, where the fragrance composition 1A is replaced by the same amount of the fragrance composition 3. Face day care cream according to table 31, where the fragrance composition 1A is replaced by the same amount of the fragrance composition 4. Face day care cream according to table 31, where the fragrance composition 1A is replaced by the same amount of the fragrance composition 5. Face day care cream according to table 31, where the fragrance composition 1A is replaced by the same amount of the fragrance composition 6.

**Table 32: Face Cleanser**

| **Ingredients** | | **INCI** | **amount [wt%]** | **Function** |
|---|---|---|---|---|
| **I** | Deionized Water | Aqua | 80.15 | |
| | EDETA B Powder (BASF) | Tetrasodium EDTA | 0.05 | Complexing Agent |
| | Eumulgin® SG | Sodium Stearoyl Glutamate | 0.30 | O/W Emulsifier |
| **II** | Cosmedia® SP | Sodium Polyacrylate | 1.20 | Emulsifier |
| **III** | Isopropylpalmitate | Isopropyl palmitate | 5.00 | Emollient |
| | Cetiol® CC | Dicaprylyl Carbonate | 3.00 | Emollient |
| | Cetiol® C5 | Coco-Caprylate | 7.00 | Emollient |
| **IV** | Plantacare® 818 UP | Coco Glucoside | 0.75 | Non Ionic Surfactant |
| **V** | Indinyl® CA LS 8998 | Water (and) Cassia Angustifolia Seed Polysaccharide | 0.50 | Smoothing and Moisturizing Active |
| | Fragrance composition 1A | Fragrance | 1.0 | Perfume |
| **VI** | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| **VII** | NaOH (Aq. Sol. 25%) | Sodium Hydroxide | 0.45 | Neutralizing Agent |

Face cleanser according to table 32, where the fragrance composition 1A is replaced by the same amount of the fragrance composition 2A. Face cleanser according to table 32, where the fragrance composition 1A is replaced by the same amount of the fragrance composition 1B. Face cleanser according to table 32, where the fragrance composition 1A is replaced by the same amount of the fragrance composition 2B. Face cleanser according to table 32, where the fragrance composition 1A is replaced by the same amount of the fragrance composition 3. Face cleanser according to table 32, where the fragrance composition 1A is replaced by the same amount of the fragrance composition 4. Face cleanser according to table 32, where the fragrance composition 1A is replaced by the same amount of the fragrance composition 5. Face cleanser according to table 32, where the fragrance composition 1A is replaced by the same amount of the fragrance composition 6.

**Table 33: Sun Care SPF50+, Sprayable Lotion**

| **Ingredients** | | **INCI / Chemistry** | **amount [wt.-%]** | **Function** |
|---|---|---|---|---|
| I. | DEHYMULS® PGPH | Polyglyceryl-2 Dipolyhydroxystearate | 4.00 | Emulsifier (W/O) |
| | CETIOL® B | Dibutyl Adipate | 8.00 | Emollient |
| | MYRITOL® 331 | Cocoglycerides | 5.00 | Emollient |
| | Tinosorb S (BASF) | Bis-Ethylhexyloxyphenol Methoxyphenyl Triazine | 3.00 | UV filter, UV-A + UV-B |
| | Uvinul T 150 ( BASF) | Ethylhexyl Triazone | 2.50 | UV filter, UV-B |
| | Uvinul A Plus (BASF) | Diethylamino Hydroxybenzoyl Hexyl Benzoate | 10.00 | UV filter, UV-A |
| | Uvinul MC 80 (BASF) | Ethylhexyl Methoxycinnamate | 10.00 | UV filter, UV-B |
| | Sensiva SC 50 (Schülke) | Ethylhexylglycerin | 0.50 | Preservative |
| II. | Water, demin. | Water | 45.55 | |
| | Glycerin | Glycerin | 3.00 | Agent, humectant |
| | EDETA BD (BASF) | Disodium EDTA | 0.05 | Chelating agent |
| | Euxyl PE 9010 (Schülke) | Phenoxyethanol, Ethylhexylglycerin | 1.00 | Preservative |
| | Keltrol CG-T (CP Kelco) | Xanthan Gum | 0.10 | Agent, thickening |
| | Veegum Ultra (RT Vanderbilt, Inc.) | Magnesium Aluminum Silicate | 2.00 | Stabilizer |
| III. | PLANTAPON® LGC SORB | Sodium Lauryl Glucose Carboxylate (and) Lauryl Glucoside | 1.50 | Surfactant |
| IV. | Eusolex T 2000 (Merck) | Titanium Dioxide and Alumina and Simethicone | 3.5 | UV-A + UV-B filter |
| V. | DN-AGE™ PW LS 9827 | Cassia Alata Leaf Extract (and) Maltodextrin | 0.10 | Active ingredient |
| | Fragrance composition 1A | Fragrance | 1.0 | Perfume |

Sun Care SPF50+, sprayable lotion according to table 33, where the fragrance composition 1A is replaced by the same amount of the fragrance composition 2A. Sun Care SPF50+, sprayable lotion according to table 33, where the fragrance composition 1A is replaced by the same amount of the fragrance composition 1B. Sun Care SPF50+, sprayable lotion according to table 33, where the fragrance composition 1A is replaced by the same amount of the fragrance composition 2B. Sun Care SPF50+, sprayable lotion according to table 33, where the fragrance composition 1A is replaced by the same amount of the fragrance composition 3. Sun Care SPF50+, sprayable lotion according to table 33, where the fragrance composition 1A is replaced by the same amount of the fragrance composition 4. Sun Care SPF50+, sprayable lotion according to table 33, where the fragrance composition 1A is replaced by the same amount of the fragrance composition 5. Sun Care SPF50+, sprayable lotion according to table 33, where the fragrance composition 1A is replaced by the same amount of the fragrance composition 6.

**Table 35: Body lotion**

| **Ingredients** | | **INCI** | **amount [wt%]** | **Function** |
|---|---|---|---|---|
| **I** | Eumulgin® SG | Sodium Stearoyl Glutamate | 0.40 | O/W Emulsifier |
| | Cutina® GMS-V | Glyceryl Stearate | 1.50 | Consistency Factor |
| | Cetiol® SB 45 | Butyrospermum Parkii (Shea) Butter | 3.00 | Emollient |
| | Cetiol® C5 | Coco-Caprylate | 2.00 | Emollient |
| | Irwinol™ LS 9890 | Octyldodecanol (and) Irvingia Gabonensis (and) Hydrogenated Coco-Glycerides | 1.00 | Moisturizing Active |
| **II** | Water, demin. | Aqua | 86.77 | |
| | Glycerine | Glycerin | 3.00 | Humectant |
| | EDETA B Powder (BASF) | Tetrasodium EDTA | 0.05 | Complexing Agent |
| **III** | Rheocare™ C Plus | Carbomer | 0.35 | Thickener |
| **IV** | NaOH (Aq sol. 25%) | Sodium Hydroxide | 0.10 | Neutralizing Agent |
| **V** | Covi-ox® T70C | Tocopherol | 0.05 | Antioxidant |
| | Fragrance composition 1A | Fragrance | 1.0 | Perfume |
| **VI** | Euxyl PE 9010 (Schülke) | Phenoxyethanol (and) Ethylhexylglycerin | 1.00 | Preservative |
| | Sensiva SC50 (Schülke) | Ethylhexylglycerin | 0.50 | Preservative |
| **VII** | NaOH (Aq sol. 25%) | Sodium Hydroxide | 0.18 | Neutralizing Agent |

Body lotion according to table 35, where the fragrance composition 1A is replaced by the same amount of the fragrance composition 1B. Body lotion according to table 35, where the fragrance composition 1A is replaced by the same amount of the fragrance composition 2A. Body lotion according to table 35, where the fragrance composition 1A is replaced by the same amount of the fragrance composition 2B. Body lotion according to table 35, where the fragrance composition 1A is replaced by the same amount of the fragrance composition 3. Body lotion according to table 35, where the fragrance composition 1A is replaced by the same amount of the fragrance composition 4. Body lotion according to table 35, where the fragrance composition 1A is replaced by the same amount of the fragrance composition 5. Body lotion according to table 35, where the fragrance composition 1A is replaced by the same amount of the fragrance composition 6.

**Table 36 Shampoo composition:**

| **Chemical name [CAS No]** | **wt.-%** |
|---|---|
| Citric acid [77-92-9] | 0.5 |
| Sodium Hydroxide [1310-73-2] | 0.7 |
| Amides, coco, N,N-bis(hydroxyethyl) [68603-42-9] | 5.5 |
| 1-Propanaminium, 3-amino-N-(carboxymethyl)-N,N-dimethyl-, N-(C8-18 and C18-unsaturated acyl) derivatives, inner salts [147170-44-3] | 7.0 |
| Fragrance Composition 1A | 1.0 |
| Water | Add 100 |

Shampoo according to table 36, where the fragrance composition 1A is replaced by the same amount of the fragrance composition 1B. Shampoo according to table 36, where the fragrance composition 1A is replaced by the same amount of the fragrance composition 2A. Shampoo according to table 36, where the fragrance composition 1A is replaced by the same amount of the fragrance composition 2B. Shampoo according to table 36, where the fragrance composition 1A is replaced by the same amount of the fragrance composition 3. Shampoo according to table 36, where the fragrance composition 1A is replaced by the same amount of the fragrance composition 4. Shampoo according to table 36, where the fragrance composition 1A is replaced by the same amount of the fragrance composition 5. Shampoo according to table 36, where the fragrance composition 1A is replaced by the same amount of the fragrance composition 6.

**Table D1: Hand dish cleaner, regular**

| **Component** | **INCI** | **amount [wt%]** |
|---|---|---|
| Lutensit ® A-LBN 50 | Benzenesulfonic acid, C10-13-alkyl derivatives, sodium salts | 25 |
| Lutensol ® GD 70 | Alkyl polyglucoside | 3 |
| Lutensol ® A 7 N | Fatty alcohol ethoxylate | 2 |
| Fragrance composition 1A | Fragrance | 0.7 |
| Dyes | Dye | 0.05 |
| Water, deionized | Aqua | ad 100 |

Hand dish cleaner according to table D1, where the fragrance composition 1A is replaced by the same amount of the fragrance composition 2A. Hand dish cleaner according to table D1, where the fragrance composition 1A is replaced by the same amount of the fragrance composition 1B. Hand dish cleaner according to table D1, where the fragrance composition 1A is replaced by the same amount of the fragrance composition 2B. Hand dish cleaner according to table D1, where the fragrance composition 1A is replaced by the same amount of the fragrance composition 3. Hand dish cleaner according to table D1, where the fragrance composition 1A is replaced by the same amount of the fragrance composition 4. Hand dish cleaner according to table D1, where the fragrance composition 1A is replaced by the same amount of the fragrance composition 5. Hand dish cleaner according to table D1, where the fragrance composition 1A is replaced by the same amount of the fragrance composition 6.

**Table D2: Hand dish cleaner, concentrate:**

| **Component** | **INCI** | **amount [wt%]** |
|---|---|---|
| Lutensit ® A-LBN 50 | Benzenesulfonic acid, C10-13-alkyl derivatives, sodium salts | 40 |
| Lutensit ® AS 2230 | Alkylether sulphate | 25 |
| Lutensol ® GD 70 | Alkyl polyglucoside | 7 |
| Lutensol ® A 7 N | Fatty alcohol ethoxylate | 4 |
| Fragrance composition 1A | Perfume | 1 |
| Dyes | Dye | 0.05 |
| Water, de ionized | Aqua | ad 100 |

Hand dish cleaner according to table D2, where the fragrance composition 1A is replaced by the same amount of the fragrance composition 2A. Hand dish cleaner according to table D2, where the fragrance composition 1A is replaced by the same amount of the fragrance composition 1B. Hand dish cleaner according to table D2, where the fragrance composition 1A is replaced by the same amount of the fragrance composition 2B. Hand dish cleaner according to table D2, where the fragrance composition 1A is replaced by the same amount of the fragrance composition 3. Hand dish cleaner according to table D2, where the fragrance composition 1A is replaced by the same amount of the fragrance composition 4. Hand dish cleaner according to table D2, where the fragrance composition 1A is replaced by the same amount of the fragrance composition 5. Hand dish cleaner according to table D2, where the fragrance composition 1A is replaced by the same amount of the fragrance composition 6.

**Table D3: Sanitary cleaner, concentrate**

| **Component** | **INCI** | **amount [wt%]** |
|---|---|---|
| Lutensol ® TO 8 | isotridecanolethoxylate | 5 |
| Phosphoric acid | Phosphoric acid | 20 |
| Korantin ® PM | 2-propyn-1-ol, ethoxylated | 3 |
| Citric acid | Citric acid | 3 |
| Fragrance composition 1A | Fragrance | 1 |
| Water, deionized | Aqua | ad 100 |

Sanitary cleaner according to table D3, where the fragrance composition 1A is replaced by the same amount of the fragrance composition 1B.Sanitary cleaner according to table D3, where the fragrance composition 1A is replaced by the same amount of the fragrance composition 2A. Sanitary cleaner according to table D3, where the fragrance composition 1A is replaced by the same amount of the fragrance composition 2B. Sanitary cleaner according to table D3, where the fragrance composition 1A is replaced by the same amount of the fragrance composition 3. Sanitary cleaner according to table D3, where the fragrance composition 1A is replaced by the same amount of the fragrance composition 4. Sanitary cleaner according to table D3, where the fragrance composition 1A is replaced by the same amount of the fragrance composition 5. Sanitary cleaner according to table D3, where the fragrance composition 1A is replaced by the same amount of the fragrance composition 6.

**Table D4: All-purpose cleaner**

| **Component** | **INCI** | **amount [wt%]** |
|---|---|---|
| Lutensit ® A-LBA | Benzenesulfonic acid, 4-C10-13-sec-alkyl derivatives | 1 |
| Ammonia | Ammonia | 0.2 |
| Lutensol ® CS 6250 | Hexan-1-ol, ethoxylated | 3 |
| Trilon ® A | trisodium nitrilotriacetate | 1 |
| Citric acid | Citric acid | 0.65 |
| Ethanol | Ethanol | 5 |
| Fragrance composition 1A | Perfume | 1.5 |
| Water, de ionized | Aqua | ad 100 |

All-purpose cleaner according to table D4, where the fragrance composition 1A is replaced by the same amount of the fragrance composition 1B. All-purpose cleaner according to table D4, where the fragrance composition 1A is replaced by the same amount of the fragrance composition 2A. All-purpose cleaner according to table D4, where the fragrance composition 1A is replaced by the same amount of the fragrance composition 2B. All-purpose cleaner according to table D4, where the fragrance composition 1A is replaced by the same amount of the fragrance composition 3. All-purpose cleaner according to table D4, where the fragrance composition 1A is replaced by the same amount of the fragrance composition 4. All-purpose cleaner according to table D4, where the fragrance composition 1A is replaced by the same amount of the fragrance composition 5. All-purpose cleaner according to table D4, where the fragrance composition 1A is replaced by the same amount of the fragrance composition 6.

**Table D5: Anti-bacterial fabric softener**

| **Component** | **INCI** | **amount [wt%]** |
|---|---|---|
| Tinosan ® HP 100 | Hydroxydichlordiphenyl ether | 0.3 |
| Dehyquat AU-46 | Esterquat | 4 |
| Lutensol ® AO 7 | Alcohols, C₁₃-₁₅, ethoxylated | 0.5 |
| Fragrance composition 1A | Fragrance | 1.0 |
| Water, deionized | Aqua | ad 100 |

Anti-bacterial fabric softener according to table D5, where the fragrance composition 1A is replaced by the same amount of the fragrance composition 1B.Anti-bacterial fabric softener according to table D5, where the fragrance composition 1A is replaced by the same amount of the fragrance composition 2A. Anti-bacterial fabric softener according to table D5, where the fragrance composition 1A is replaced by the same amount of the fragrance composition 2B. Anti-bacterial fabric softener according to table D5, where the fragrance composition 1A is replaced by the same amount of the fragrance composition 3. Anti-bacterial fabric softener according to table D5, where the fragrance composition 1A is replaced by the same amount of the fragrance composition 4. Anti-bacterial fabric softener according to table D5, where the fragrance composition 1A is replaced by the same amount of the fragrance composition 5. Anti-bacterial fabric softener according to table D5, where the fragrance composition 1A is replaced by the same amount of the fragrance composition 6.

**Table D6: Detergent composition 1**

| **Ingredient** [CAS No] | **wt.-%** |
|---|---|
| MEA-C10-13 ALKYL BENZENESULFONATE [85480-55-3] | 12.4 |
| MEA-COCOATE [66071-80-5] | 7.5 |
| C14-15 PARETH-7 [68951-67-7] | 5.7 |
| Ethanol [64-17-5] | 2 |
| Fragrance Composition 1A | 1.0 |
| water | balance to 100 g |

Detergent composition according to table D6, where the fragrance composition 1A is replaced by the same amount of the fragrance composition 1B. Detergent composition according to table D6, where the fragrance composition 1A is replaced by the same amount of the fragrance composition 2A. Detergent composition according to table D6, where the fragrance composition 1A is replaced by the same amount of the fragrance composition 2B. Detergent composition according to table D6, where the fragrance composition 1A is replaced by the same amount of the fragrance composition 3. Detergent composition according to table D6, where the fragrance composition 1A is replaced by the same amount of the fragrance composition 4. Detergent composition according to table D6, where the fragrance composition 1A is replaced by the same amount of the fragrance composition 5. Detergent composition according to table D6, where the fragrance composition 1A is replaced by the same amount of the fragrance composition 6.

**Table D7: Detergent composition 2**

| **Ingredient** | **[wt.-% of solids]** |
|---|---|
| n-C₁₂H₂₅-O-(CH₂CH₂O)₃-SO₃Na | 20.2 |
| n-C₁₈H₃₇-O(CH₂CH₂O)₇-H | 20.2 |
| sodium salt of linear dodecylbenzenesulphonate | 20.6 |
| coconut soap, K salt | 9 |
| Fragrance Composition 1A | 0.8 |
| pH value | 8.5 |
| Total solids content (%) | 26.7 |

All amounts in g/100 g of solids content of the respective formulation.

Detergent composition according to table D7, where the fragrance composition 1A is replaced by the same amount of the fragrance composition 1B. Detergent composition according to table D7, where the fragrance composition 1A is replaced by the same amount of the fragrance composition 2A. Detergent composition according to table D7, where the fragrance composition 1A is replaced by the same amount of the fragrance composition 2B. Detergent composition according to table D7, where the fragrance composition 1A is replaced by the same amount of the fragrance composition 3. Detergent composition according to table D7, where the fragrance composition 1A is replaced by the same amount of the fragrance composition 4. Detergent composition according to table D7, where the fragrance composition 1A is replaced by the same amount of the fragrance composition 5. Detergent composition according to table D7, where the fragrance composition 1A is replaced by the same amount of the fragrance composition 6.

**Table D8: Detergent compositions 3A 3B and 3C**

| **Detergent composition** | **3A** | **3B** | **3C** |
|---|---|---|---|
| **Ingredient [in g]** | | | |
| n-C₁₀-C₁₃-alkylbenzene sulfonic acid | 10.4 | 17,9 | 13 |
| coconut C₁₂-C₁₈ fatty acid | 2.5 | 8.5 | 8.5 |
| potassium hydroxide | 3.4 | 5 | 4.4 |
| C₁₃C₁₅ oxo alcohol ethoxylate (7 EO) | 5.7 | 20 | 7.5 |
| 1,2 propyleneglycol | 6 | 8 | 8 |
| Sodium citrate - dihydrate | - | 3 | 3 |
| Ethanol | 2 | 2 | 2 |
| Fragrance Composition 1A | 1.0 | 1.0 | 1.0 |
| Additional KOH | to pH 9 | to pH 8.6 | to pH 8.6 |
| Water | balance to 100 g | balance to 100 g | balance to 100 g |

Detergent composition according to composition 3A of table D8, where the fragrance composition 1A is replaced by the same amount of the fragrance composition 1B. Detergent composition according to composition 3A of table D8, where the fragrance composition 1A is replaced by the same amount of the fragrance composition 2A. Detergent composition according to composition 3A of table D8, where the fragrance composition 1A is replaced by the same amount of the fragrance composition 2B. Detergent composition according to composition 3A of table D8, where the fragrance composition 1A is replaced by the same amount of the fragrance composition 3. Detergent composition according to composition 3A of table D8, where the fragrance composition 1A is replaced by the same amount of the fragrance composition 4. Detergent composition according to composition 3A of table D8, where the fragrance composition 1A is replaced by the same amount of the fragrance composition 5. Detergent composition according to composition 3A of table D8, where the fragrance composition 1A is replaced by the same amount of the fragrance composition 6.

Detergent composition according to composition 3B of table D8, where the fragrance composition 1A is replaced by the same amount of the fragrance composition 1B. Detergent composition according to composition 3B of table D8, where the fragrance composition 1A is replaced by the same amount of the fragrance composition 2A. Detergent composition according to composition 3B of table D8, where the fragrance composition 1A is replaced by the same amount of the fragrance composition 2B. Detergent composition according to composition 3B of table D8, where the fragrance composition 1A is replaced by the same amount of the fragrance composition 3. Detergent composition according to composition 3B of table D8, where the fragrance composition 1A is replaced by the same amount of the fragrance composition 4. Detergent composition according to composition 3B of table D8, where the fragrance composition 1A is replaced by the same amount of the fragrance composition 5. Detergent composition according to composition 3B of table D8, where the fragrance composition 1A is replaced by the same amount of the fragrance composition 6.

Detergent composition according to composition 3C of table D8, where the fragrance composition 1A is replaced by the same amount of the fragrance composition 1B. Detergent composition according to composition 3C of table D8, where the fragrance composition 1A is replaced by the same amount of the fragrance composition 2A. Detergent composition according to composition 3C of table D8, where the fragrance composition 1A is replaced by the same amount of the fragrance composition 2B. Detergent composition according to composition 3C of table D8, where the fragrance composition 1A is replaced by the same amount of the fragrance composition 3. Detergent composition according to composition 3C of table D8, where the fragrance composition 1A is replaced by the same amount of the fragrance composition 4. Detergent composition according to composition 3C of table D8, where the fragrance composition 1A is replaced by the same amount of the fragrance composition 5. Detergent composition according to composition 3C of table D8, where the fragrance composition 1A is replaced by the same amount of the fragrance composition 6.

**Table D9: Powder detergent composition:**

| **Ingredient** | |
|---|---|
| Linear sodium alkyl benzene sulfonate | 9.7 % |
| Ethoxylated fatty alcohol C12-18 (7 EO) | 5.2 % |
| Sodium soap | 3.6 % |
| Anti-foam DC2-4248S | 24.5 % |
| Sodium aluminium silicate zeolite 4A | 32.5 % |
| Sodium carbonate | 11.8 % |
| Sodium salt of a copolymer from acrylic and maleic acid | 5.2% |
| Sodium silicate (SiO2:Na2O = 3,3:1) | 3.4 % |
| Carboxymethylcellulose | 1.3 % |
| Diethylene triamine penta (methylene phosphonic acid) | 0.8 % |
| Sodium sulfate | 9.8 % |
| Fragrance Composition 1A | 1.0 % |
| Water | 12.2 % |

Detergent composition according to table D9, where the fragrance composition 1A is replaced by the same amount of the fragrance composition 1B. Detergent composition according to table D9, where the fragrance composition 1A is replaced by the same amount of the fragrance composition 2A. Detergent composition according to table D9, where the fragrance composition 1A is replaced by the same amount of the fragrance composition 2B. Detergent composition according to table D9, where the fragrance composition 1A is replaced by the same amount of the fragrance composition 3. Detergent composition according to table D9, where the fragrance composition 1A is replaced by the same amount of the fragrance composition 4. Detergent composition according to table D9, where the fragrance composition 1A is replaced by the same amount of the fragrance composition 5. Detergent composition according to table D9, where the fragrance composition 1A is replaced by the same amount of the fragrance composition 6.

**Table D10 Detergent compositions 10A, 10B, 10C and 10D**

| | Composition, % by weight | | | |
|---|---|---|---|---|
| Components | 10A | 10B | 10C | 10D |
| Linear C₁₀C₁₃-alkylbenzene sulfonates, sodium salt | 6.0 | 2.0 | 4.0 | 4.8 |
| Sodium laureth sulfate + 2 mole ethylene oxide | - | 4.5 | 16.0 | 14.4 |
| Alkyl ether ethoxylate (7 EO): | 24.0 | - | - | - |
| C₁₂C₁₄-fatty alcohol + 7 mole ethylene oxide | | | | |
| Alkyl ether ethoxylate (9 EO): | - | 17.5 | 4.0 | 4.8 |
| C₁₂C₁₄ fatty alcohol + 9 mole ethylene oxide | | | | |
| Sodium citrate | 0.5 | 0.1 | - | 0.1 |
| Mono propylene glycol | 5.0 | 1.0 | - | 1.0 |
| Ethanol | 1.5 | - | - | - |
| Toluene sulfonic acid | - | - | - | 0.2 |
| Fragrance Composition 1A | 1.0 | 1.0 | 1.0 | 1.0 |
| Deionized water | Add to 100 | | | |

Detergent composition according to composition 10A of table D10, where the fragrance composition 1A is replaced by the same amount of the fragrance composition 1B. Detergent composition according to composition 10A of table D10, where the fragrance composition 1A is replaced by the same amount of the fragrance composition 2A. Detergent composition according to composition 10A of table D10, where the fragrance composition 1A is replaced by the same amount of the fragrance composition 2B. Detergent composition according to composition 10A of table D10, where the fragrance composition 1A is replaced by the same amount of the fragrance composition 3. Detergent composition according to composition 10A of table D10, where the fragrance composition 1A is replaced by the same amount of the fragrance composition 4. Detergent composition according to composition 10A of table D10, where the fragrance composition 1A is replaced by the same amount of the fragrance composition 5. Detergent composition according to composition 10A of table D10, where the fragrance composition 1A is replaced by the same amount of the fragrance composition 6. Detergent composition according to composition 10B of table D10, where the fragrance composition 1A is replaced by the same amount of the fragrance composition 1B. Detergent composition according to composition 10B of table D10, where the fragrance composition 1A is replaced by the same amount of the fragrance composition 2A. Detergent composition according to composition 10B of table D10, where the fragrance composition 1A is replaced by the same amount of the fragrance composition 2B. Detergent composition according to composition 10B of table D10, where the fragrance composition 1A is replaced by the same amount of the fragrance composition 3. Detergent composition according to composition 10B of table D10, where the fragrance composition 1A is replaced by the same amount of the fragrance composition 4. Detergent composition according to composition 10B of table D10, where the fragrance composition 1A is replaced by the same amount of the fragrance composition 5. Detergent composition according to composition 10B of table D10, where the fragrance composition 1A is replaced by the same amount of the fragrance composition 6. Detergent composition according to composition 10C of table D10, where the fragrance composition 1A is replaced by the same amount of the fragrance composition 1B. Detergent composition according to composition 10C of table D10, where the fragrance composition 1A is replaced by the same amount of the fragrance composition 2A. Detergent composition according to composition 10C of table D10, where the fragrance composition 1A is replaced by the same amount of the fragrance composition 2B. Detergent composition according to composition 10C of table D10, where the fragrance composition 1A is replaced by the same amount of the fragrance composition 3. Detergent composition according to composition 10C of table D10, where the fragrance composition 1A is replaced by the same amount of the fragrance composition 4. Detergent composition according to composition 10C of table D10, where the fragrance composition 1A is replaced by the same amount of the fragrance composition 5. Detergent composition according to composition 10C of table D10, where the fragrance composition 1A is replaced by the same amount of the fragrance composition 6. Detergent composition according to composition 10D of table D10, where the fragrance composition 1A is replaced by the same amount of the fragrance composition 1B. Detergent composition according to composition 10D of table D10, where the fragrance composition 1A is replaced by the same amount of the fragrance composition 2A. Detergent composition according to composition 10D of table D10, where the fragrance composition 1A is replaced by the same amount of the fragrance composition 2B. Detergent composition according to composition 10D of table D10, where the fragrance composition 1A is replaced by the same amount of the fragrance composition 3. Detergent composition according to composition 10D of table D10, where the fragrance composition 1A is replaced by the same amount of the fragrance composition 4. Detergent composition according to composition 10D of table D10, where the fragrance composition 1A is replaced by the same amount of the fragrance composition 5. Detergent composition according to composition 10D of table D10, where the fragrance composition 1A is replaced by the same amount of the fragrance composition 6.

**Table D11: Detergent composition DC1**

| Ingredients | percentage by weight |
|---|---|
| Alkyl Benzene sulfonate¹ | 7.50% |
| AE3S² | 2.60% |
| AE9³ | 0.40% |
| NI 45-7⁴ | 4.40% |
| Citric Acid | 3.20% |
| C12-18 Fatty acid | 3.10% |
| Amphiphilic polymer⁵ | 0.50% |
| Zwitterionic dispersant⁶ | 1.00% |
| Ethoxylated Polyethyleneimine⁷ | 1.51% |
| Protease⁸ | 0.89% |
| Enymes⁹ | 0.21% |
| Chelant¹⁰ | 0.28% |
| Brightener¹¹ | 0.09% |
| Solvent | 7.35% |
| Sodium Hydroxide | 3.70% |
| Fragrance composition 1A | 1.0% |
| Water, filler, stucturant | to Balance |

| | |
|---|---|
| ¹ Linear alkylbenenesulfonate having an average aliphatic carbon chain length C11-C12 supplied by Stepan, Northfield Illinois, USA // ²AE3S is C12-15 alkyl ethoxy (3) sulfate supplied by Stepan, USA // ^{3B}E9 is C12-14 alcohol ethoxylate, with an average degree of ethoxylation of 9, supplied by Huntsman, Salt Lake City, Utah, USA // ⁴ NI 45-7 is C14-15 alcohol ethoxylate, with an average degree of ethoxylation of 7, supplied by Huntsman, USA, //⁵ Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. // ⁶ A compound having the following general structure: bis((C2H5O)(C2H4O)n)(CH3)-N+-CxH2x-N+-(CH3)-bis((C2H5O)(C2H4O)n), wherein n = from 20 to 30, and x = from 3 to 8, or sulphated or sulphonated variants thereof //⁷ Polyethyleneimine (MW = 600) with 20 ethoxylate groups per -NH // ⁸ Proteases may be supplied by Genencor International, Palo Alto, California, USA (e.g. Purafect Prime®) or by Novozymes, Bagsvaerd, Denmark (e.g. Liquanase®, Coronase®). //⁹ Natalase®, Mannaway® are all products of Novozymes, Bagsvaerd, Denmark. //¹⁰ Suitable chelants are, for example, diethylenetetraamine pentaacetic acid (DTPA) supplied by Dow Chemical, Midland, Michigan, USA or Hydroxyethane di phosphonate (HEDP) or diethylene triamine penta(methyl phosphonic) acid supplied by Solutia, St Louis, Missouri, USA; // ¹¹ Fluorescent Brightener 1 is Tinopal® AMS, Fluorescent Brightener 2 supplied by Ciba Specialty Chemicals, Basel, Switzerland | |

Detergent composition according to table D11, where the fragrance composition 1A is replaced by the same amount of the fragrance composition 1B. Detergent composition according to table D11, where the fragrance composition 1A is replaced by the same amount of the fragrance composition 2A. Detergent composition according to table D11, where the fragrance composition 1A is replaced by the same amount of the fragrance composition 2B. Detergent composition according to table D11, where the fragrance composition 1A is replaced by the same amount of the fragrance composition 3. Detergent composition according to table D11, where the fragrance composition 1A is replaced by the same amount of the fragrance composition 4. Detergent composition according to table D11, where the fragrance composition 1A is replaced by the same amount of the fragrance composition 5. Detergent composition according to table D11, where the fragrance composition 1A is replaced by the same amount of the fragrance composition 6.

**Table D12: Liquid detergent composition**

| **Ingredient** | **Wt.-%** |
|---|---|
| AES C₁₂₋₁₅ alkyl ethoxy (1.8) sulfate | 10.9 |
| Linear alkylbenzenesulfonate having an average aliphatic carbon chain length C11-C12 supplied by Stepan, Northfield, Illinois, USA | 1.56 |
| Sodium formate | 2.66 |
| Sodium hydroxide | 0.21 |
| Monoethanolamine (MEA) | 1.65 |
| Diethylene glycol (DEG) | 4.10 |
| AE9 (C₁₂₋₁₃ alcohol ethoxylate, with an average degree of ethoxylation of 9) | 0.40 |
| diethylenetetraamine pentaacetic acid (DTPA) | 0.18 |
| Citric Acid | 1.70 |
| C₁₂₋₁₈ Fatty Acid | 1.47 |
| Borax | 1.19 |
| Ethanol | 1.44 |
| Polyethyleneimine (MW = 600) with 20 ethoxylate groups per -NH | 1.35 |
| A compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof | 0.40 |
| 1,2-Propanediol | 2.40 |
| Protease (54.5 mg active/g) | 0.89 |
| Mannanase: Mannaway® (25.6 mg active/g) | 0.04 |
| Amylase: Natalase® (29 mg active/g) | 0.14 |
| Fluorescent Whitening Agent: Sulphonated zinc phthalocyanine | 0.10 |
| Fragrance composition 1A | 1.0 |
| Water | Ad 100 |

| | |
|---|---|
| Natalase®, Mannaway® are all products of Novozymes, Bagsvaerd, Denmark. Proteases may be supplied by Genencor International, Palo Alto, California, USA (e.g. Purafect Prime®) or by Novozymes, Bagsvaerd, Denmark (e.g. Liquanase®, Coronase®). | |

Detergent composition according to table D12, where the fragrance composition 1A is replaced by the same amount of the fragrance composition 1B. Detergent composition according to table D12, where the fragrance composition 1A is replaced by the same amount of the fragrance composition 2A. Detergent composition according to table D12, where the fragrance composition 1A is replaced by the same amount of the fragrance composition 2B. Detergent composition according to table D12, where the fragrance composition 1A is replaced by the same amount of the fragrance composition 3. Detergent composition according to table D12, where the fragrance composition 1A is replaced by the same amount of the fragrance composition 4. Detergent composition according to table D12, where the fragrance composition 1A is replaced by the same amount of the fragrance composition 5. Detergent composition according to table D12, where the fragrance composition 1A is replaced by the same amount of the fragrance composition 6.

**Table D13: Powder Detergent Compositions 13A, 13B, 13C:**

| | 13A (wt.-%) | 13B (wt.-%) | 13C (wt.-%) |
|---|---|---|---|
| Linear alkylbenzenesulfonatehaving an average aliphatic carbon chain length C₁₁-C₁₂ | 8.2 | 8.2 | 8.2 |
| AE3S (=C12-15 alkyl ethoxy (3) sulfate supplied by Stepan) | 1.9 | 1.9 | 1.9 |
| Zeolite A (supplied by Industrial Zeolite (UK)) | 1.8 | 1.8 | 1.8 |
| Citric Acid | 1.5 | 1.5 | 1.5 |
| Sodium Carbonate (supplied by Solvay, USA) | 29.7 | 29.7 | 29.7 |
| Silicate 1.6R (SiO₂:Na₂O) (supplied by Koma, Czech Rep) | 3.4 | 3.4 | 3.4 |
| Soil release agent: Repel-o-tex® PF 8 (supplied by Rhodia, France | 0.2 | 0.2 | 0.2 |
| Acrylic Acid/Maleic Acid Copolymer (molecular weight 70,000 and acrylate:maleate ratio 70:30, supplied by BASF) | 2.2 | 2.2 | 2.2 |
| Carboxymethylcellulose | 0.9 | 0.9 | 0.9 |
| Protease - Purafect® (84 mg active/g) | 0.08 | 0.08 | 0.08 |
| Amylase - Stainzyme Plus® (20 mg active/g) | 0.16 | 0.16 | 0.16 |
| Lipase - Lipex® (18.00 mg active/g) | 0.24 | 0.24 | 0.24 |
| Cellulase - Celluclean™ (15.6 mg active/g) | 0.1 | 0.1 | 0.1 |
| TAED (tetraacetylethylenediamine, supplied under the Per-active® brand name by Clariant GmbH) | 3.26 | 3.26 | 3.26 |
| Percarbonate | 14.1 | 14.1 | 14.1 |
| Na salt of Ethylenediamine-N,N'-disuccinic acid, (S,S) isomer (EDDS) supplied by Octel, Ellesmere Port, UK | 2.19 | 2.19 | 2.19 |
| Hydroxyethane di phosphonate (HEDP), supplied by Dow Chemical | 0.54 | 0.54 | 0.54 |
| MgSO₄ | 0.38 | 0.38 | 0.38 |
| Perfume | 0.38 | 0.38 | 0.38 |
| Suds suppressor agglomerate (supplied by Dow Corning) | 0.04 | 0.04 | 0.04 |
| Sulphonated zinc phthalocyanine (active) Tinopal® AMS | 0.0012 | - | - |
| Sulphonated zinc phthalocyanine (active) Tinopal® CBS-X | - | 0.0012 | - |
| Dye Pergasol® Violet BN-Z | - | - | 0.012 |
| Fragrance Composition 1A | 1.0 | 1.0 | 1.0 |
| Sulfate/ Water & Miscellaneous | Balance | Balance | Balance |

| | | | |
|---|---|---|---|
| Savinase®, Natalase®, Stainzyme®, Lipex®, Celluclean™, Mannaway® and Whitezyme® are all products of Novozymes, Bagsvaerd, Denmark. Proteases may be supplied by Genencor International, Palo Alto, California, USA (e.g. Purafect Prime®) or by Novozymes, Bagsvaerd, Denmark (e.g. Liquanase®, Coronase®). | | | |

Detergent composition according to composition 13A of table D13, where the fragrance composition 1A is replaced by the same amount of the fragrance composition 1B. Detergent composition according to composition 13A of table D13, where the fragrance composition 1A is replaced by the same amount of the fragrance composition 2A. Detergent composition according to composition 13A of table D13, where the fragrance composition 1A is replaced by the same amount of the fragrance composition 2B. Detergent composition according to composition 13A of table D13, where the fragrance composition 1A is replaced by the same amount of the fragrance composition 3. Detergent composition according to composition 13A of table D13, where the fragrance composition 1A is replaced by the same amount of the fragrance composition 4. Detergent composition according to composition 13A of table D13, where the fragrance composition 1A is replaced by the same amount of the fragrance composition 5. Detergent composition according to composition 13A of table D13, where the fragrance composition 1A is replaced by the same amount of the fragrance composition 6. Detergent composition according to composition 13B of table D13, where the fragrance composition 1A is replaced by the same amount of the fragrance composition 1B. Detergent composition according to composition 13B of table D13, where the fragrance composition 1A is replaced by the same amount of the fragrance composition 2A. Detergent composition according to composition 13B of table D13, where the fragrance composition 1A is replaced by the same amount of the fragrance composition 2B. Detergent composition according to composition 13B of table D13, where the fragrance composition 1A is replaced by the same amount of the fragrance composition 3. Detergent composition according to composition 13B of table D13, where the fragrance composition 1A is replaced by the same amount of the fragrance composition 4. Detergent composition according to composition 13B of table D13, where the fragrance composition 1A is replaced by the same amount of the fragrance composition 5. Detergent composition according to composition 13B of table D13, where the fragrance composition 1A is replaced by the same amount of the fragrance composition 6. Detergent composition according to composition 13C of table D13, where the fragrance composition 1A is replaced by the same amount of the fragrance composition 1B. Detergent composition according to composition 13C of table D13, where the fragrance composition 1A is replaced by the same amount of the fragrance composition 2A. Detergent composition according to composition 13C of table D13, where the fragrance composition 1A is replaced by the same amount of the fragrance composition 2B. Detergent composition according to composition 13C of table D13, where the fragrance composition 1A is replaced by the same amount of the fragrance composition 3. Detergent composition according to composition 13C of table D13, where the fragrance composition 1A is replaced by the same amount of the fragrance composition 4. Detergent composition according to composition 13C of table D13, where the fragrance composition 1A is replaced by the same amount of the fragrance composition 5. Detergent composition according to composition 13C of table D13, where the fragrance composition 1A is replaced by the same amount of the fragrance composition 6.

## Claims

1. Use of a compound of formula (I), 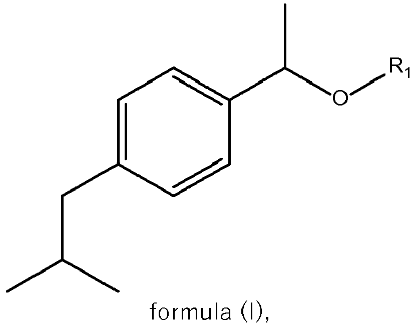 wherein
R₁ is selected from the group consisting of linear or branched, unsubstituted -C₁-C₅ alkyl and unsubstituted -C₃-C₅ cycloalkyl;
or its salt or stereoisomer thereof,
to impart an aroma impression to a composition.

2. Method of imparting an aroma impression to a composition comprising
at least the step of adding a compound of formula (I), 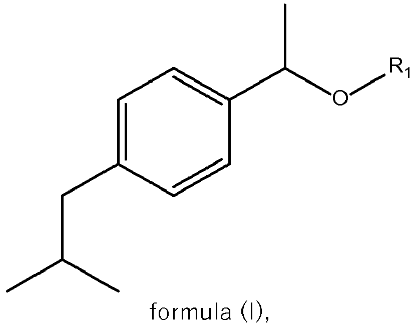 wherein
R₁ is selected from the group consisting of linear or branched, unsubstituted -C₁-C₅ alkyl and unsubstituted -C₃-C₅ cycloalkyl;
or its salt or stereoisomer thereof,
to a composition.

3. The use or method according to any of the preceding claims, wherein R₁ is selected from the group consisting of -CH₃, -CH₂CH₃, -CH₂CH₂CH₃, -CH(CH₃)₂, -CH(CH₃)(C₂H₅), -CH₂CH(CH₃)₂, -CH₂CH₂CH₂CH₃ and cyclopropyl.

4. The use or method according to any of the preceding claims, wherein the composition selected from perfume compositions, body care compositions, hygiene articles, cleaning compositions, textile detergent compositions, compositions for scent dispensers, foods, food supplements, pharmaceutical compositions and crop protection compositions.

5. The use or method according to any of the preceding claims, wherein the impression is selected from a group consisting of a flowery note, a marine note, a sweet note, a rubbery note, a nutty note, a woody note, a dusty note, a rooty note, a lemon note and a green note.

6. The use or method according to any of the preceding claims, wherein the compound of formula (I) or its salts or stereoisomer is present in an amount in the range of ≥ 0.01 wt.% to ≤ 70.0 wt.%, based on the total weight of the composition.

7. A composition comprising a at least one compound of the formula (I), 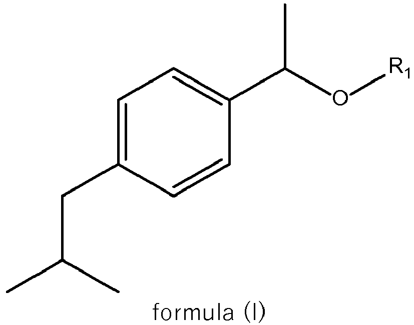 wherein
R₁ is selected from the group consisting of linear or branched, unsubstituted -C₁-C₅ alkyl and unsubstituted -C₃-C₅ cycloalkyl;
or its salt or stereoisomer thereof and
(i) at least one aroma chemical, or
(ii) at least one non-aroma chemical carrier, or
(iii) both of (i) and (ii).

8. The composition according to claim 8, wherein R₁ is selected from the group consisting of -CH₃, -CH₂CH₃, -CH₂CH₂CH₃, -CH(CH₃)₂, -CH(CH₃)(C₂H₅), -CH₂CH(CH₃)₂,-CH₂CH₂CH₂CH₃, cyclopropyl and cyclobutyl.

9. The composition according to any one of the claims 7 or 8, wherein the at least one non-aroma chemical carrier (ii) is selected from the group consisting of surfactants, oil components, anti-oxidants, deodorant-active agents and solvents.

10. The composition according to any of the preceding claims 7 to 9, wherein the composition is selected from the group consisting of perfume compositions, body care compositions, hygiene articles, cleaning compositions, textile detergent compositions, compositions for scent dispensers, foods, food supplements, pharmaceutical compositions and crop protection compositions.

11. A compound of formula (I'), 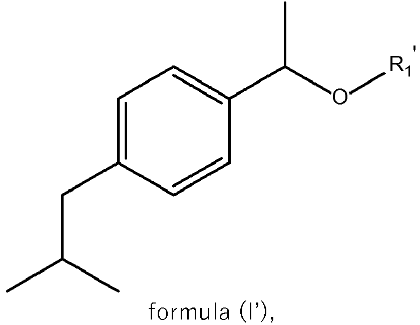 wherein
R₁' is selected from the group consisting of linear or branched -CH₂CH₂CH₃,-CH(CH₃)(C₂H₅), -CH₂CH(CH₃)₂ and -CH₂CH₂CH₂CH₃;
or its salt or stereoisomer thereof.
